# EUROPEAN PATENT APPLICATION

(11) **EP 2 489 373 A1**
(43) Date of publication of application: **22.08.2012**
(21) Application number: 12150409.6
(22) Date of filing: 14.05.2002
(51) Int. Cl.: A61L 9/18, A61L 9/20, A61L 9/16, B01D 53/86, B01D 53/88, B01J 35/00, B01J 35/06, B60H 3/00, B60H 3/06, F24F 3/16

(54) **Photocatalyst deodorizer**

(30) Priority: 14.06.2001 JP 2001179786; 03.09.2001 JP 2001265758
(62) Divisional of application: 02780777.5
(71) Applicant: Zexel Valeo Climate Control Corporation, Ohsato-gun, Saitama 360-0193 (JP)
(72) Inventor: Hara, Shinichi, Ohsato-gun, Saitama 360-0193 (JP)
(74) Representative: Vieillevigne, Sébastien Nicolas Bernard

(57) **Abstract**

Object of this invention is to provide a photocatalyst deodorizer wherein it is possible to extend the life and increasing an efficiency of filters, and it is possible to activate a photocatalyst which a deodorizing filter carries, by optimizing the positional relation between a tubular light source for emitting light to activatee a photocatalyst and a deodorizing filter, and an emission angle of the tubular light source. Further a photocatalyst deodorizer of this invention makes it easy to maintenance and replacement of filters and the tubular light source.

A photocatalyst deodorizer comprising an air purifying portion which is removably arranged in an air passage and constructed from a dust collecting filter, a deodorizing filter carrying a photocatalyst, and a tubular light source arranged in that order in a stack, or constructed from said dust collecting filter, said tubular light source and said deodorizing filter arranged in that order in a stack; wherein said tubular light source is arranged at a position which obtains total surface irradiation of one side of said deodorizing filter, and set at an emission angle which obtains total surface irradiation.

## Description

### TECHNICAL FIELD

The present invention is related to a photocatalyst deodorizer and a filter unit thereof having an air purification function which carries out purification by removing contaminants in indoor air and a deodorizing function which depends on a photocatalyst, and in particular to a photocatalyst deodorizer installed inside an air conditioner for a vehicle.

### PRIOR ART TECHNOLOGY

Air purifiers have been developed to remove odors and dust inside a vehicle, but there is a desire to make these small and thin. In Japanese Laid-Open Patent Publication Number HEI 9-322933, a cylindrical lamp is arranged on a side of a deodorizing filter, and the apparatus is made thin in the direction of the airflow path. However, in the technology of this same publication, because a space is required for arranging the cylindrical lamp on a side portion of the deodorizing filter, the apparatus becomes large. Further, the airflow can be easily bypassed (short-circuited) between the deodorizing filter and the cylindrical lamp, and this lowers the dust collecting effect. Japanese Laid-Open Patent Publication Number HEI 11-314017 discloses a thin type air purifier in which ultraviolet light in the range 360 - 400nm from a light emitting diode is shone onto a photocatalyst, namely, a titanium oxide catalyst to serve as a deodorizing technology. In this apparatus, dust is collected before deodorizing.

Further, Japanese Laid-Open Patent Publication Number HEI 9-253451 discloses an air purifier for an automobile in which an electrodeless discharge lamp is made to emit light by microwaves, whereby ultraviolet light is shone onto a photocatalyst to oxidize carbon monoxide by a CO oxidizing catalyst. In this same publication, a filter folded to have a pleated shape with a thickness of 50mm is used, wherein activated carbon fibers are made to carry a Pd catalyst or a titanium oxide catalyst to serve as an adsorbent, and the permeation rate of air is lowered to reduce the pressure loss.

Further, Japanese Laid-Open Patent Publication Number 2001-35287 has a disclosure related to an air conditioner for a vehicle, namely, discloses technology which uses a lamp apparatus disclosed in this same publication, wherein a carrier member which carries a photocatalyst is made to function as a catalyst. In this same publication, the lamp apparatus and the adsorbent member are formed as a unit which can be easily installed in and removed from an air conditioner body.

### SUMMARY OF THE INVENTION

When a filter is folded in a pleated shape to increase the filter surface area, it is believed that the life of the filter is extended, and the dust collecting efficiency and the deodorizing efficiency are raised. However, even though on the one hand it is believed that folding a filter in a pleated shape will extend the dust collecting filter life and improve the dust collecting efficiency, it is difficult to shine the light that activates the photocatalyst on the total surface of a deodorizing filter in which peaks and valleys are formed due to the pleats. Accordingly, there are places in the deodorizing filter that do not receive light irradiation and can not carry out sufficient catalytic action, and this lowers the deodorizing efficiency. Further, when a deodorizing filter is folded in a pleated shape, the thickness of the filter is increased, and because a space for arranging a light irradiation source is secured, the direction of the airflow path becomes thick. Accordingly, it is difficult to design the miniaturization of the entire deodorizer while ensuring a long filter life and high dust collecting and deodorizing efficiencies.

It is a first object of the present invention to provide a photocatalyst deodorizer in which a dust collecting filter and a deodorizing filter are processed to have pleated shapes, and which makes it possible to sufficiently activate a photocatalyst carried in the deodorizing filter while extending the life and improving the efficiency of each filter by optimizing the positional relationship of the deodorizing filter and a tubular light source which shines light to activate the photocatalyst and the emission angle of the tubular light source. Further, this object includes providing a photocatalyst deodorizer in which maintenance and replacement of parts of the dust collecting filter, the deodorizing filter and the tubular light source can be carried out easily.

It is a second object of the present invention to provide a photocatalyst deodorizer having an optimum positional relationship of a tubular light source and a deodorizing filter. Namely, it is an object to make it possible to shine light uniformly on all portions of the deodorizing filter folded in a pleated shape without the tubular light source obstructing the airflow.

It is a third object of the present invention to provide a photocatalyst deodorizer having an even higher deodorizing efficiency by forming a structure in which a dust collecting filter, a deodorizing filter, a tubular light source and a second deodorizing filter processed to have a pleated shape and carrying a photocatalyst are arranged in an ordered stack as an air purifying portion toward the downstream side of the airflow path.

It is a fourth object of the present invention to provide a photocatalyst deodorizer which, in addition to obtaining apparatus characteristics such as the creation of a long life and the creation of a high efficiency of the dust collecting filter and the deodorizing filter, and sufficient activation of the photocatalyst carried in the deodorizing filter, also achieves miniaturization of the apparatus by a large reduction of the required space of the tubular light source. Further, this object includes providing a photocatalyst deodorizer in which maintenance such as replacement of parts and the like of the dust collecting filter, the deodorizing filter and the tubular light source can be carried out easily.

It is a fifth object of the present invention to provide a photocatalyst deodorizer which makes it possible to raise the catalytic activity and achieve a high deodorizing efficiency while keeping the size of the apparatus small by arranging a tubular light source at each of both end portions of the deodorizing filter in the direction of the peaks and valleys of the pleats, and irradiating every part of the deodorizing filter folded in a pleated shape with light at or above a prescribed intensity.

It is a sixth object of the present invention to provide a photocatalyst deodorizer which, in addition to obtaining apparatus characteristics such as the creation of a long life and the creation of a high efficiency of the dust collecting filter and the deodorizing filter, and sufficient activation of the photocatalyst carried in the deodorizing filter, is provided with a second deodorizing filter for the purpose of increasing the deodorizing filter surface area, and wherein the enlargement of the apparatus is kept to a minimum by a large reduction of the required space of the tubular light source even with the addition of the second deodorizing filter. Further, this object includes providing a photocatalyst deodorizer in which maintenance such as replacement of parts and the like of the dust collecting filter, the deodorizing filter and the tubular light source can be carried out easily.

It is a seventh object of the present invention to provide a photocatalyst deodorizer in which a second deodorizing filter is provided, and a tubular light source is provided at each of both end portions of one or both of the deodorizing filter and the second deodorizing filter in the direction of the peaks and valleys of the pleats, whereby the filter surface area is increased and every part of the deodorizing filter folded in a pleated shape is irradiated with light at or above a prescribed intensity to raise the catalytic activity and further improve the deodorizing performance, and on the other hand, which keeps enlargement of the apparatus to a minimum by a large reduction of the required space of the tubular light source.

It is an eighth object of the present invention to provide a specific embodiment which prevents the occurrence of short-circuiting of the airflow, and which is related to a depression formed for the purpose of making a large reduction of the required space of the tubular light source. Further, this object includes providing a photocatalyst deodorizer which prevents reduction of the filter surface area by making the depression portion also serve as a filter.

It is a ninth object of the present invention to provide a photocatalyst deodorizer which does not use mercury by using a xenon external electrode lamp as the tubular light source. A photocatalyst lamp uses a mercury lamp, a cathode tube (CFL), a black light or the like which use mercury, and a cold cathode tube (CCFL) is the most widespread of these. However, in recent years the use of mercury has decreased due to the problem of environmental pollution, and the same correspondence has also become necessary for ultraviolet lamps. Further, a xenon external electrode lamp can not structurally achieve a 360° total angular emission, but because the emission angle of the light can be adjusted, irradiation can be carried out efficiently on only the deodorizing filter, whereby it is also possible to prevent other parts from being degraded by ultraviolet light irradiation. Namely, the ninth object of the present invention is to provide a photocatalyst deodorizer which efficiently irradiates only the deodorizing filter without using mercury, and can prevent the degrading of other parts due to ultraviolet light irradiation.

It is a tenth object of the present invention to provide a compact photocatalyst deodorizer in which the total surface of the deodorizing filter is irradiated with light and the required space is minimized by making the length of the tubular light source roughly the same as the width of the deodorizing filter.

It is an eleventh object of the present invention to provide a photocatalyst deodorizer which, in addition to obtaining apparatus characteristics such as the creation of a long life and the creation of a high efficiency of the dust collecting filter and the deodorizing filter, and sufficient activation of the photocatalyst carried in the deodorizing filter, also achieves miniaturization of the apparatus by irradiating the deodorizing filter using a light source which can be made thin and has a small required space in which light-emitting diode elements are arranged. In Japanese Laid-Open Patent Publication Number HEI 11-314017, light-emitting diodes which do not use mercury are used as an ultraviolet light source, but because the ultraviolet light intensity of one light-emitting diode is weak, many light-emitting diodes need to be lined up. Namely, in the present invention, it is an object to provide a photocatalyst deodorizer which can achieve reliable irradiation of light to the deodorizing filter while minimizing the number of light-emitting diode elements that are used by optimizing the positional relationship of the deodorizing filter and the light-emitting diode elements. Further, this object includes providing a photocatalyst deodorizer in which maintenance such as replacement of parts and the like of the dust collecting filter, the deodorizing filter and the light source can be carried out easily.

It is a twelfth object of the present invention to provide a compact photocatalyst deodorizer having a different embodiment than that described above which uses light-emitting photodiode elements as a light source.

Incidentally, after the deodorizing filter has adsorbed odorants, because the odorants are decomposed by the photocatalyst, the life of the deodorizing filter is extended. On the other hand, the life of the dust collecting filter which can be extended by enlarging the surface area by folding in a pleated shape is not extended compared to the deodorizing filter. In this regard, when the dust collecting filter and the deodorizing filter are made to be separate bodies, because replacement can be carried out separately in accordance with the life of each filter, such arrangement is convenient and efficient. In this regard, it is a thirteenth obj ect of the present invention to provide a photocatalyst deodorizer in which the dust collecting filter and the deodorizing filter are separate bodies with one of the dust collecting filter being replaceable, and which makes it possible to achieve the same thin structure as the case where these filters are integrated even though the dust collecting filter and the deodorizing filter are made separate bodies by processing the pleated shape of the dust collecting filter so that the dust collecting filter and the deodorizing filter match multiple layers, and arranging the dust collecting filter in a stack removable with respect to the deodorizing filter. Further, it is possible to reduce the frequency of replacement of the deodorizing filter, and this leads to a reduction of the expenditures of the owner of the vehicle.

Further an object of this invention is to provide a filter unit of the photocatalyst deodorizer.

The photocatalyst deodorizer according to Claim 1 includes an air purifying portion which is removably arranged in an air passage which forms an airflow from an air intake port toward a purified air discharge port, and constructed from a dust collecting filter processed to have a pleated shape, a deodorizing filter processed to have a pleated shape and carrying a photocatalyst, and a tubular light source which emits light that activates said photocatalyst arranged in that order in a stack, or constructed from said dust collecting filter, said tubular light source and said deodorizing filter arranged in that order in a stack, wherein said tubular light source is a xenon external electrode lamp which is constructed to emit light by removing a portion of a fluorescent material applied to the inside of a glass tube which is a dielectric, said xenon external electrode lamp is arranged near one end portion of said deodorizing filter in a corner portion which does not obstruct the airflow of said airflow passage so that the tube axis direction thereof forms a direction which intersects the pleats of said deodorizing filter, and the angle of an aperture formed by removing fluorescent material of said xenon external electrode lamp is set to roughly match an emission angle which obtains total surface irradiation of one side of said deodorizing filter.

Preferably, in the photocatalyst deodorizer described in Claim 1, said air purifying portion is constructed from said dust collecting filter, said deodorizing filter, said tubular light source and a second deodorizing filter processed to have a pleated shape and carrying a photocatalyst arranged in that order in a stack toward the downstream side of said air passage, said tubular light source is a xenon external electrode lamp which is constructed to emit light by removing a portion of a fluorescent material applied to the inside of a glass tube which is a dielectric, said xenon external electrode lamp is arranged near one end portion of said deodorizing filter and the facing one side of said second deodorizing filter in a corner portion which does not obstruct the airflow of said airflow passage so that the tube axis direction thereof forms a direction which intersects the pleats of said deodorizing filter and said second deodorizing filter, and the angle of an aperture formed by removing fluorescent material of said xenon external electrode lamp is set to roughly match an emission angle which obtains total surface irradiation of each of one side of said deodorizing filter and a facing one side of said second deodorizing filter.

The photocatalyst deodorizer according to Claim 4 includes an air purifying portion which is removably arranged in an air passage which forms an airflow from an air intake port toward a purified air discharge port, and constructed from a dust collecting filter processed to have a pleated shape, a deodorizing filter processed to have a pleated shape and carrying a photocatalyst, and a tubular light source which emits light that activates said photocatalyst arranged in that order in a stack, or constructed from said dust collecting filter, said tubular light source and said deodorizing filter arranged in that order in a stack, wherein said tubular light source is a xenon external electrode lamp which is constructed to emit light by removing a portion of a fluorescent material applied to the inside of a glass tube which is a dielectric, a depression capable of housing said xenon external electrode lamp is provided in said deodorizing filter in a direction which intersects the peaks and valleys of the pleats, said xenon external electrode lamp is arranged inside said depression at a position which obtains total surface irradiation of one side of said deodorizing filter, and the angle of an aperture formed by removing fluorescent material of said xenon external electrode lamp is set to roughly match an emission angle which obtains total surface irradiation of one side of said deodorizing filter.
The depression in the present invention is for housing the tubular light source in order to make the distance between the deodorizing filter and the tubular light source extremely close. Accordingly, the size and depth of the depression need to be in a range which makes it possible to house the tubular light source and have sufficient size, and if the size is small or the depth is shallow, the tubular light source can not be housed, and on the other hand, if the size is large or the depth is deep, the filter surface area will be reduced and the total surface of one side of the deodorizing filter can not be irradiated with light. Further, it is important that the depression of the present invention be a depression that does not have a bypass portion which creates air leaks. When a bypass portion is created due to the formation of a depression, the opening thereof is occluded, or the depression needs to be formed by folding the pleats without creating an opening. This is because a lowering of the deodorizing efficiency will occur if a bypass portion exists.

Preferably, in the photocatalyst deodorizer described in Claim 4, a depression capable of housing said tubular light source is provided in both end portions of said deodorizing filter in the direction of the peaks and valleys of the pleats, and a tubular light source is arranged inside each depression to obtain total surface irradiation of one side of said deodorizing filter.

The photocatalyst deodorizer according to Claim 6 includes an air purifying portion which is removably arranged in an air passage which forms an airflow from an air intake port toward a purified air discharge port, and constructed from a dust collecting filter processed to have a pleated shape, a deodorizing filter processed to have a pleated shape and carrying a photocatalyst, a tubular light source which emits light that activates said photocatalyst, and a second deodorizing filter processed to have a pleated shape and carrying a photocatalyst arranged in that order in a stack, wherein said tubular light source is a xenon external electrode lamp which is constructed to emit light by removing a portion of a fluorescent material applied to the inside of a glass tube which is a dielectric, a depression capable of housing said xenon external electrode lamp is provided in one or both of said deodorizing filter and said second deodorizing filter in a direction which intersects the peaks and valleys of the pleats, said xenon external electrode lamp is arranged inside said depression at a position which obtains total surface irradiation of each of one side of said deodorizing filter and a facing one side of said second deodorizing filter, and the angle of an aperture formed by removing fluorescent material of said xenon external electrode lamp is set to roughly match an emission angle which obtains total surface irradiation of each of one side of said deodorizing filter and a facing one side of said second deodorizing filter.

Preferably, in the photocatalyst deodorizer described in Claim 6, a depression capable of housing said tubular light source is provided in both end portions of one or both of said deodorizing filter and said second deodorizing filter in the direction of the peaks and valleys of the pleats, and said tubular light source is arranged inside said depression to obtain total surface irradiation of one side of said deodorizing filter and a facing one side of said second deodorizing filter.

Preferably, in the photocatalyst deodorizer described in Claim 4, 5, 6 or 7, said depression is formed to have a notch shape with an occluded opening or is formed by folding the pleats.

Preferably, in the photocatalyst deodorizer described in Claim 1, 3, 4, 5, 6, 7, or 8, said tubular light source has roughly the same length as the width of said deodorizing filter.

The photocatalyst deodorizer according to Claim 11 includes an air purifying portion which is removably arranged in an air passage which forms an airflow from an air intake port toward a purified air discharge port, and constructed from a dust collecting filter processed to have a pleated shape, a deodorizing filter processed to have a pleated shape and carrying a photocatalyst, and a light source which emits light that activates said photocatalyst arranged in that order in a stack, or constructed from said dust collecting filter, said light source and said deodorizing filter arranged in that order in a stack, wherein said light source is constructed from light-emitting diode elements provided in roughly a line on top of a strip-shaped substrate at spacings where said light-emitting diode elements are arranged at each valley of the pleats of said deodorizing filter, and is arranged to obtain total surface irradiation of one side of said deodorizing filter.

The photocatalyst deodorizer according to Claim 12 includes an air purifying portion which is removably arranged in an air passage which forms an airflow from an air intake port toward a purified air discharge port, and constructed from a dust collecting filter processed to have a pleated shape and a deodorizing filter processed to have a pleated shape and carrying a photocatalyst arranged in that order in a stack, and a light source which emits light that activates the photocatalyst arranged closely at both end portions of said deodorizing filter in the direction of the peaks and valleys of the pleats, wherein each of said light source is constructed from light-emitting diode elements provided in roughly a line on top of a strip-shaped substrate at spacings where said light-emitting diode elements are arranged at each valley of the pleats of said deodorizing filter, and is arranged to obtain total surface irradiation of one side of said deodorizing filter.

Preferably, in the photocatalyst deodorizer described in Claim 1, 3, 4, 5, 6, 7, 8, 10, 11 or 12, the pleated shape of said dust collecting filter is processed to match multiple layers with said deodorizing filter, and said dust collecting filter is arranged to be removably stacked with respect to said deodorizing filter.

Preferably, in the photocatalyst deodorizer described in Claim 1, 10 or 13, when there is a plurality of the tubular light source described Claim 1, at least one of said tubular light sources is arranged near one end portion of said deodorizing filter in a corner portion which does not obstruct the airflow of said airflow passage so that the tube axis direction thereof forms a direction which intersects the pleats of said deodorizing filter.

Preferably, in the photocatalyst deodorizer described in Claim 3, 10 or 13, when there is a plurality of the tubular light source described Claim 3, at least one of said tubular light sources is arranged near one end portion of said deodorizing filter and the facing one side of said second deodorizing filter in a corner portion which does not obstruct the airflow of said airflow passage so that the tube axis direction thereof forms a direction which intersects the pleats of said deodorizing filter and said second deodorizing filter.

The filter unit described in Claim 16, which is removably arranged in an air passage of a photocatalyst deodorizer which forms an airflow from an air intake port toward a purified air discharge port, comprising a structure formed from a dust collecting filter processed to have a pleated shape, a deodorizing filter processed to have a pleated shape and carrying a photocatalyst, and a tubular light source which emits light that activates said photocatalyst arranged in that order in a stack, or a structure formed from said dust collecting filter, said tubular light source and said deodorizing filter arranged in that order in a stack, wherein said tubular light source is a xenon external electrode lamp which is constructed to emit light by removing a portion of a fluorescent material applied to the inside of a glass tube which is a dielectric, said xenon external electrode lamp is arranged near one end portion of said deodorizing filter in a corner portion which does not obstruct the airflow of said airflow passage so that the tube axis direction thereof forms a direction which intersects the pleats of said deodorizing filter, and the angle of an aperture formed by removing fluorescent material of said xenon external electrode lamp is set to roughly match an emission angle which obtains total surface irradiation of one side of said deodorizing filter.

The filter unit described in Claim 17, which is removably arranged in an air passage of a photocatalyst deodorizer which forms an airflow from an air intake port toward a purified air discharge port, comprising a structure formed from a dust collecting filter processed to have a pleated shape, a deodorizing filter processed to have a pleated shape and carrying a photocatalyst, and a tubular light source which emits light that activates said photocatalyst arranged in that order in a stack, or a structure formed from said dust collecting filter, said tubular light source and said deodorizing filter arranged in that order in a stack, wherein said tubular light source is a xenon external electrode lamp which is constructed to emit light by removing a portion of a fluorescent material applied to the inside of a glass tube which is a dielectric, said xenon external electrode lamp is arranged near one end portion of said deodorizing filter and supported by an end portion of a unit frame so that the tube axis direction thereof forms a direction which intersects the pleats of said deodorizing filter, and the angle of an aperture formed by removing fluorescent material of said xenon external electrode lamp is set to roughly match an emission angle which obtains total surface irradiation of one side of said deodorizing filter.

The filter unit described in Claim 18, which is removably arranged in an air passage of a photocatalyst deodorizer which forms an airflow from an air intake port toward a purified air discharge port, comprising a structure formed from a dust collecting filter processed to have a pleated shape, a deodorizing filter processed to have a pleated shape and carrying a photocatalyst, a tubular light source which emits light that activates said photocatalyst, and a second deodorizing filter processed to have a pleated shape and carrying a photocatalyst arranged in that order in a stack, wherein said tubular light source is a xenon external electrode lamp which is constructed to emit light by removing a portion of a fluorescent material applied to the inside of a glass tube which is a dielectric, said xenon external electrode lamp is arranged near one end portion of said deodorizing filter and the facing one side of said second deodorizing filter in a corner portion which does not obstruct the airflow of said airflow passage so that the tube axis direction thereof forms a direction which intersects the pleats of said deodorizing filter and said second deodorizing filter, and the angle of an aperture formed by removing fluorescent material of said xenon external electrode lamp is set to roughly match an emission angle which obtains total surface irradiation of each of one side of said deodorizing filter and a facing one side of said second deodorizing filter.

The filter unit described in Claim 19, which is removably arranged in an air passage of a photocatalyst deodorizer which forms an airflow from an air intake port toward a purified air discharge port, comprising a structure formed from a dust collecting filter processed to have a pleated shape, a deodorizing filter processed to have a pleated shape and carrying a photocatalyst, a tubular light source which emits light that activates said photocatalyst, and a second deodorizing filter processed to have a pleated shape and carrying a photocatalyst arranged in that order in a stack, wherein said tubular light source is a xenon external electrode lamp which is constructed to emit light by removing a portion of a fluorescent material applied to the inside of a glass tube which is a dielectric, said xenon external electrode lamp is arranged near one end portion of said deodorizing filter and the facing one side of said second deodorizing filter and supported by an end portion of a unit frame so that the tube axis direction thereof forms a direction which intersects the pleats of said deodorizing filter and said second deodorizing filter, and the angle of an aperture formed by removing fluorescent material of said xenon external electrode lamp is set to roughly match an emission angle which obtains total surface irradiation of each of one side of said deodorizing filter and a facing one side of said second deodorizing filter.

The filter unit described in Claim 20, which is removably arranged in an air passage of a photocatalyst deodorizer which forms an airflow from an air intake port toward a purified air discharge port, comprising a structure formed from a dust collecting filter processed to have a pleated shape, a deodorizing filter processed to have a pleated shape and carrying a photocatalyst, and a tubular light source which emits light that activates said photocatalyst arranged in that order in a stack, or a structure formed from said dust collecting filter, said tubular light source and said deodorizing filter arranged in that order in a stack, wherein said tubular light source is a xenon external electrode lamp which is constructed to emit light by removing a portion of a fluorescent material applied to the inside of a glass tube which is a dielectric, a depression capable of housing said xenon external electrode lamp is provided in said deodorizing filter in a direction which intersects the peaks and valleys of the pleats, said xenon external electrode lamp is arranged inside said depression at a position which obtains total surface irradiation of one side of said deodorizing filter, and the angle of an aperture formed by removing fluorescent material of said xenon external electrode lamp is set to roughly match an emission angle which obtains total surface irradiation of one side of said deodorizing filter. Preferably, the filter unit, wherein a depression capable of housing said tubular light source is provided in both end portions of said deodorizing filter in the direction of the peaks and valleys of the pleats, and a tubular light source is arranged inside each depression to obtain total surface irradiation of one side of said deodorizing filter.

The filter unit described in Claim 22, which is removably arranged in an air passage of a photocatalyst deodorizer which forms an airflow from an air intake port toward a purified air discharge port, comprising a structure formed from a dust collecting filter processed to have a pleated shape, a deodorizing filter processed to have a pleated shape and carrying a photocatalyst, a tubular light source which emits light that activates said photocatalyst, and a second deodorizing filter processed to have a pleated shape and carrying a photocatalyst arranged in that order in a stack, wherein said tubular light source is a xenon external electrode lamp which is constructed to emit light by removing a portion of a fluorescent material applied to the inside of a glass tube which is a dielectric, a depression capable of housing said xenon external electrode lamp is provided in one or both of said deodorizing filter and said second deodorizing filter in a direction which intersects the peaks and valleys of the pleats, said xenon external electrode lamp is arranged inside said depression at a position which obtains total surface irradiation of each of one side of said deodorizing filter and a facing one side of said second deodorizing filter, and the angle of an aperture formed by removing fluorescent material of said xenon external electrode lamp is set to roughly match an emission angle which obtains total surface irradiation of each of one side of said deodorizing filter and a facing one side of said second deodorizing filter. Preferably, the filter unit described in Claim 22, wherein a depression capable of housing said tubular light source is provided in both end portions of one or both of said deodorizing filter and said second deodorizing filter in the direction of the peaks and valleys of the pleats, and a tubular light source is arranged inside each depression to obtain total surface irradiation of each of one side of said deodorizing filter and a facing one side of said second deodorizing filter.

Preferably, in the filter unit described in Claim 20, 21, 22 or 23, said depression is formed to have a notch shape with an occluded opening or is formed by folding the pleats.

Preferably, in the filter unit described in Claim 16, 17, 18, 19, 20, 21, 22, 23 or 24, said tubular light source has roughly the same length as the width of said deodorizing filter.

The filter unit described in Claim 26, which is removably arranged in an air passage of a photocatalyst deodorizer which forms an airflow from an air intake port toward a purified air discharge port, comprising a structure formed from a dust collecting filter processed to have a pleated shape, a deodorizing filter processed to have a pleated shape and carrying a photocatalyst, and a tubular light source which emits light that activates said photocatalyst arranged in that order in a stack, or a structure formed from said dust collecting filter, said tubular light source and said deodorizing filter arranged in that order in a stack, wherein said light source is constructed from light-emitting diode elements provided in roughly a line on top of a strip-shaped substrate at spacings where said light-emitting diode elements are arranged at each valley of the pleats of said deodorizing filter, and is arranged to obtain total surface irradiation of one side of said deodorizing filter.

The filter unit described in Claim 27, which is removably arranged in an air passage of a photocatalyst deodorizer which forms an airflow from an air intake port toward a purified air discharge port, comprising a structure formed from a dust collecting filter processed to have a pleated shape and a deodorizing filter processed to have a pleated shape and carrying a photocatalyst arranged in that order in a stack, and a light source which emits light that activates the photocatalyst arranged closely at both end portions of said deodorizing filter in the direction of the peaks and valleys of the pleats, wherein each of said light source is constructed from light-emitting diode elements provided in roughly a line on top of a strip-shaped substrate at spacings where said light-emitting diode elements are arranged at each valley of the pleats of said deodorizing filter, and is arranged to obtain total surface irradiation of one side of said deodorizing filter.

Preferably, in the filter unit described in Claim 16, 17, 18, 19, 20, 21, 22, 23, 24, 25, 26 or 27, the pleated shape of said dust collecting filter is processed to match multiple layers with said deodorizing filter, and said dust collecting filter is arranged to be removably stacked with respect to said deodorizing filter.

Preferably, in the filter unit described in Claim 16, 18, 20, 21, 22, 23, 24, 25, 26, 27 or 28, the tubular light source is supported by an end portion of a unit frame.

The photocatalyst deodorizers described in Claims 1 ∼ 29 make it possible to achieve the object stated above.

### BRIEF DESCRIPTION OF THE DRAWINGS

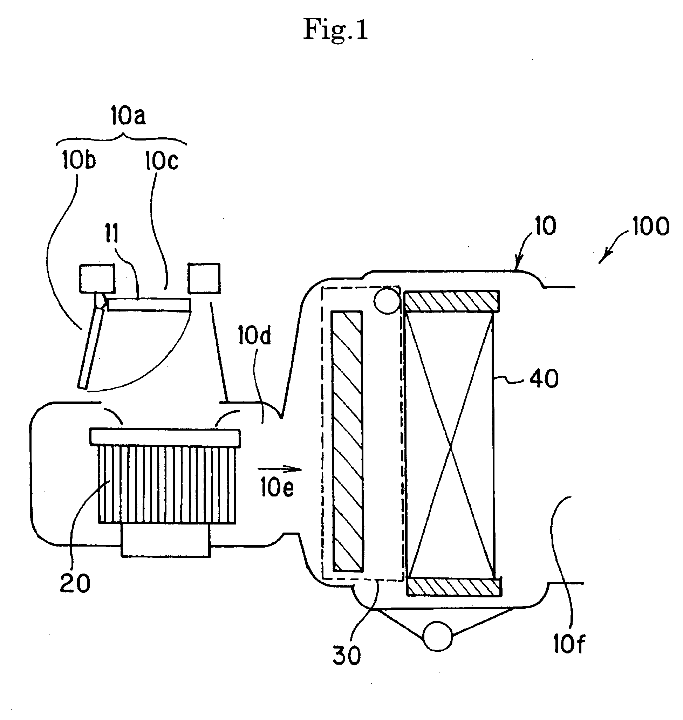

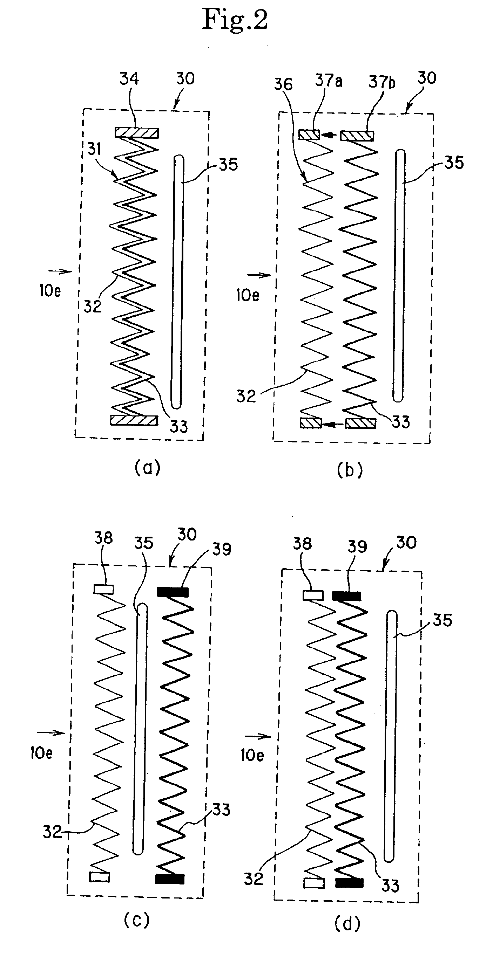

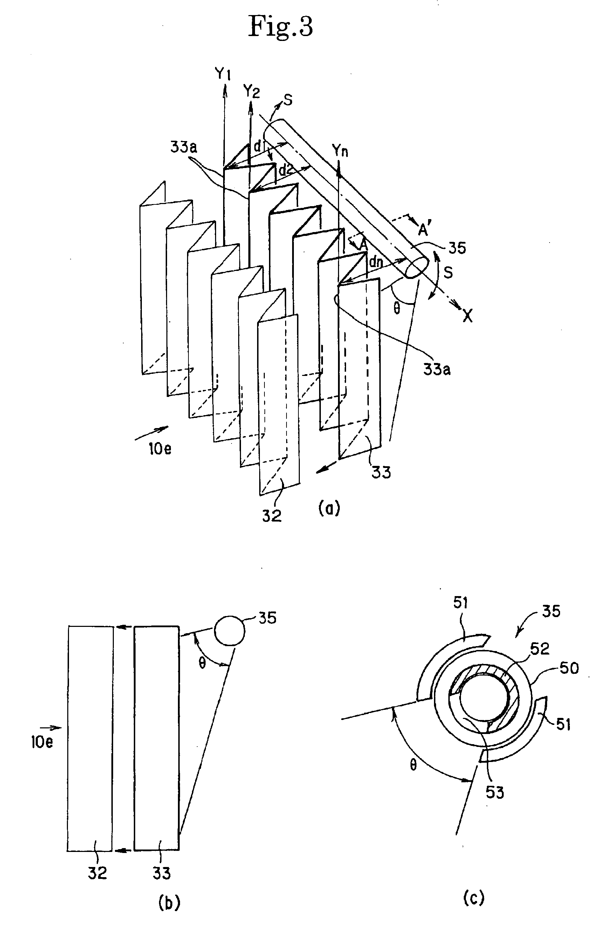

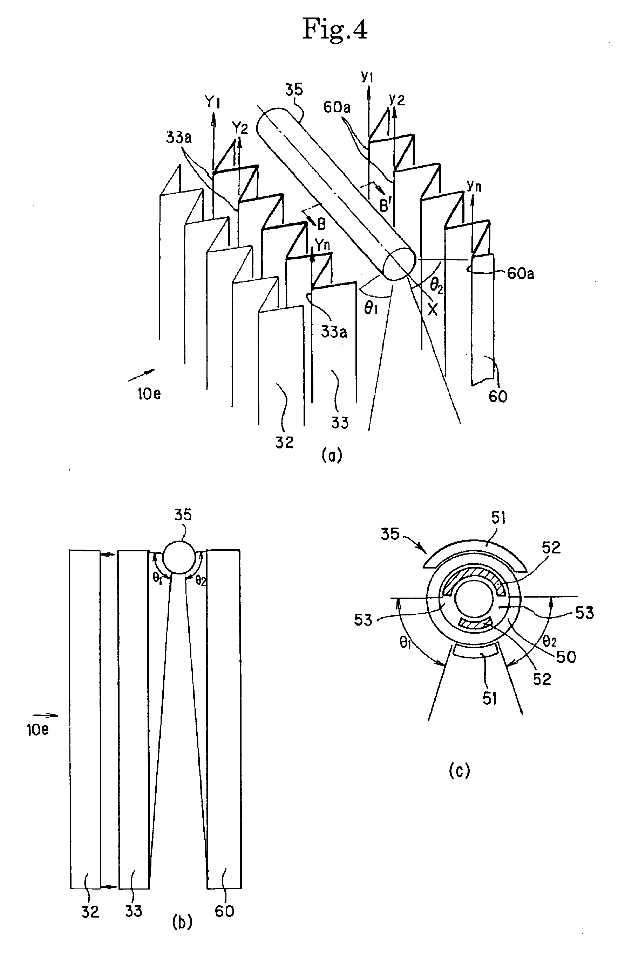

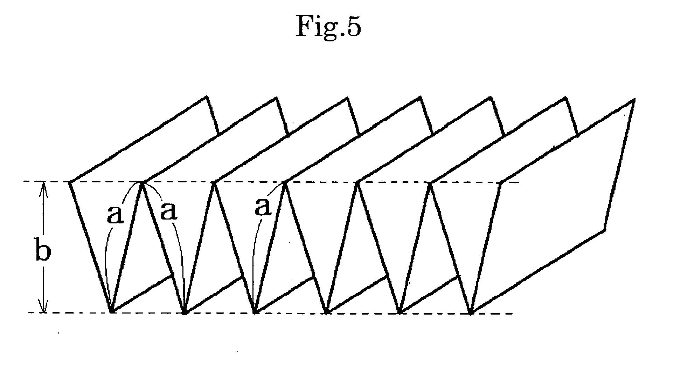

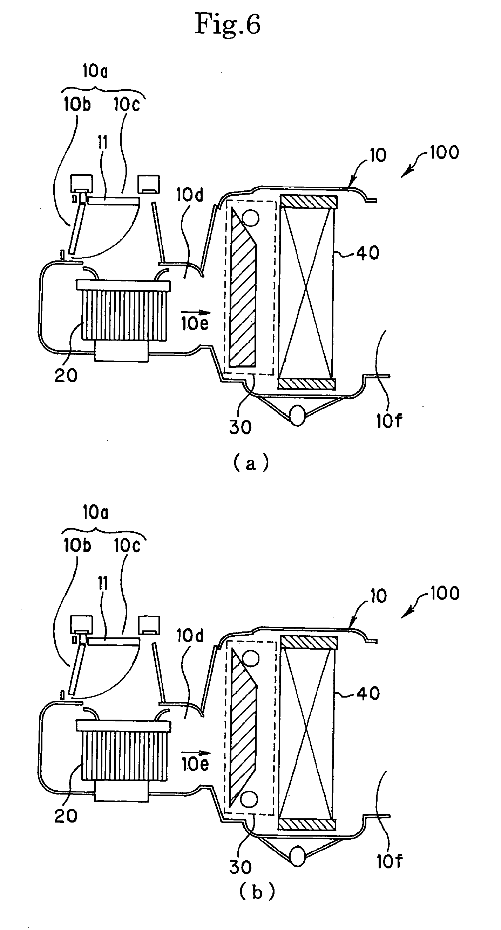

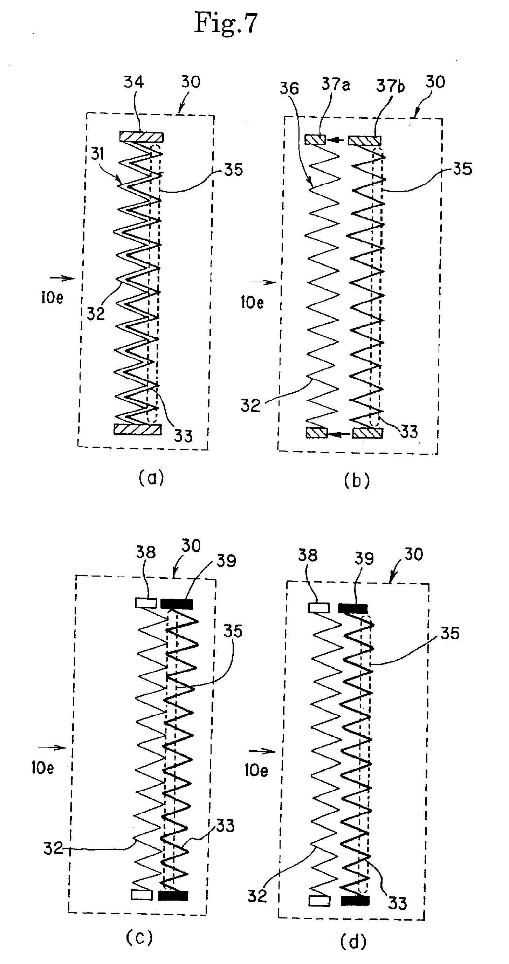

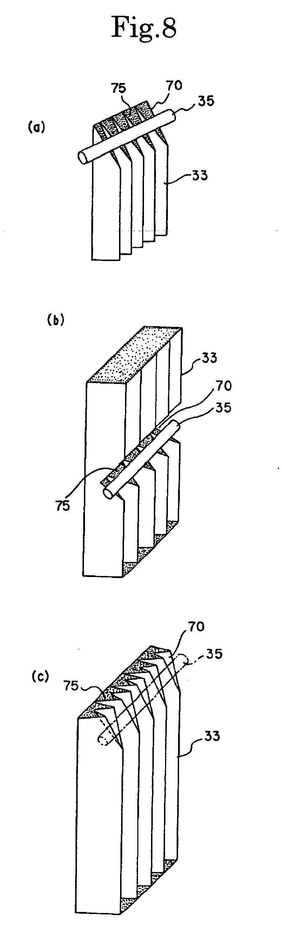

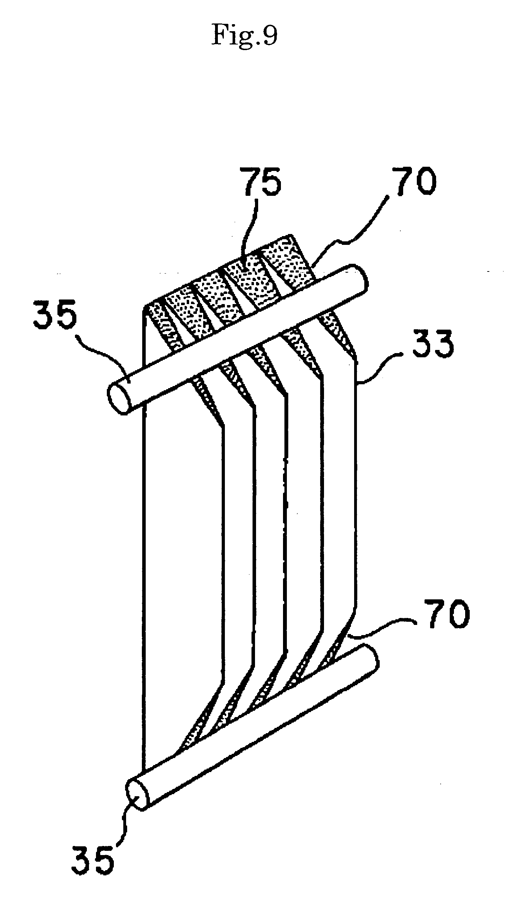

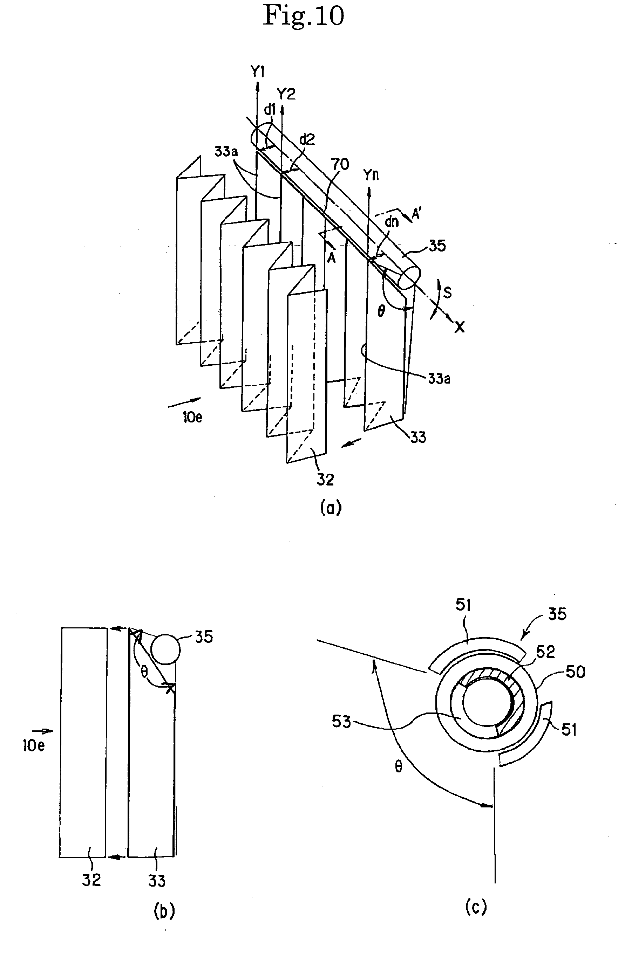

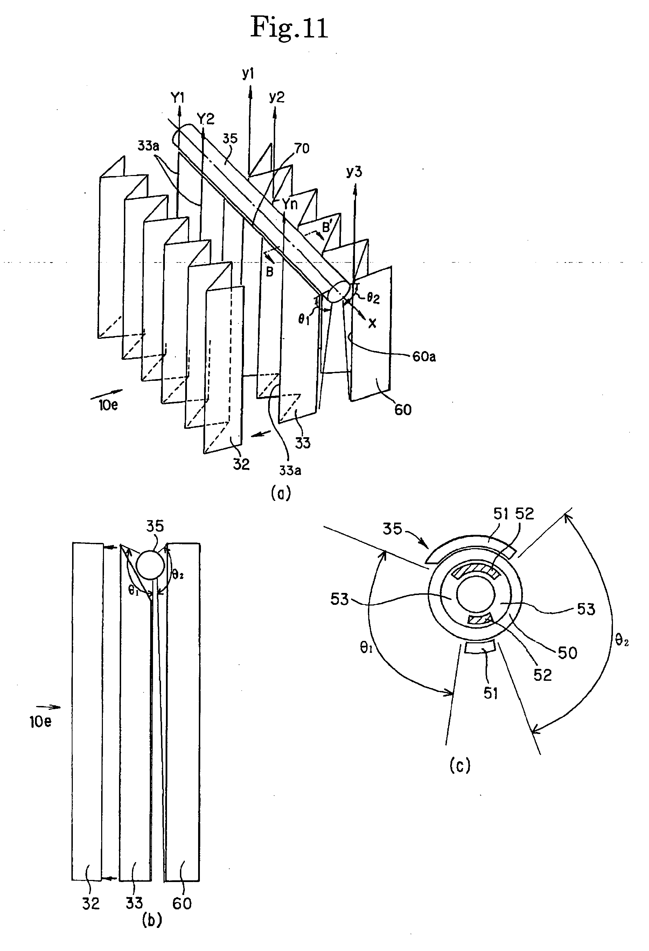

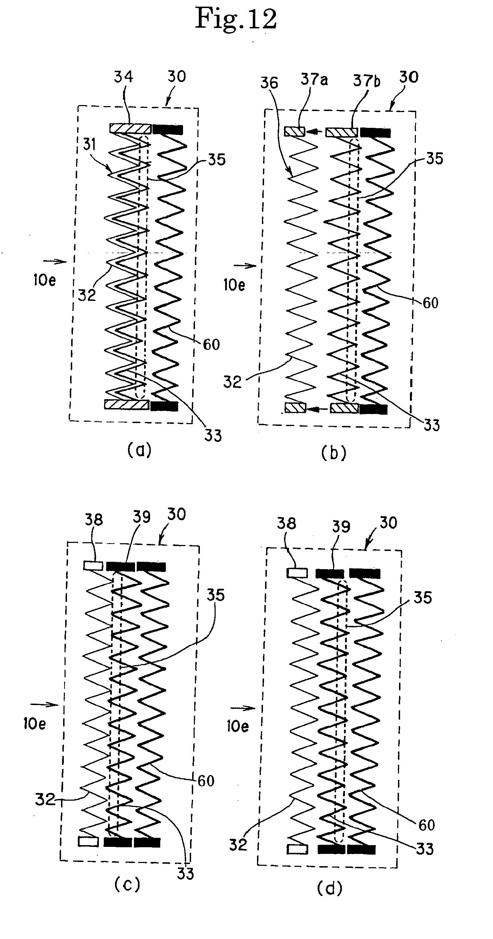

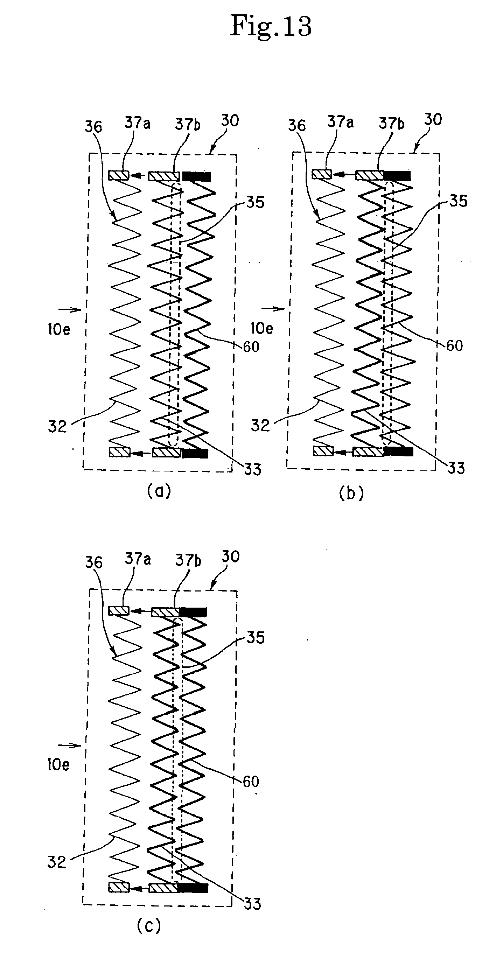

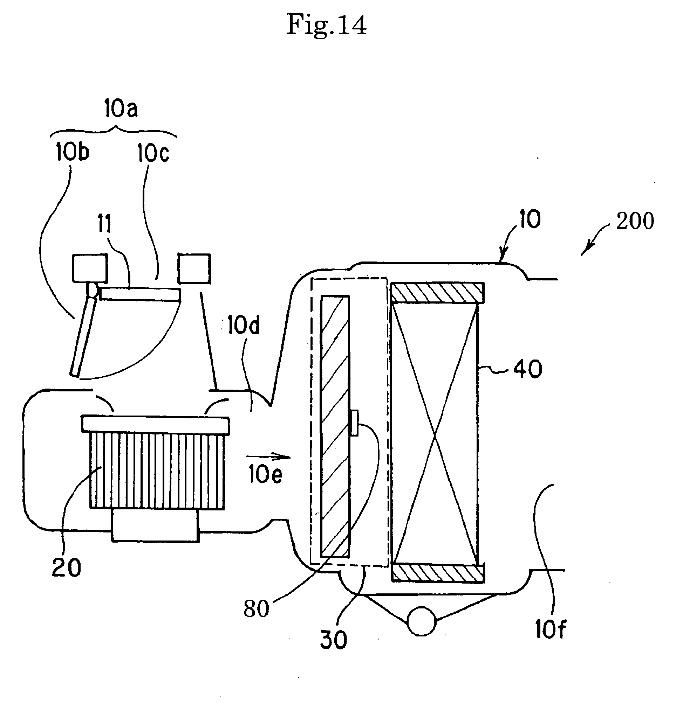

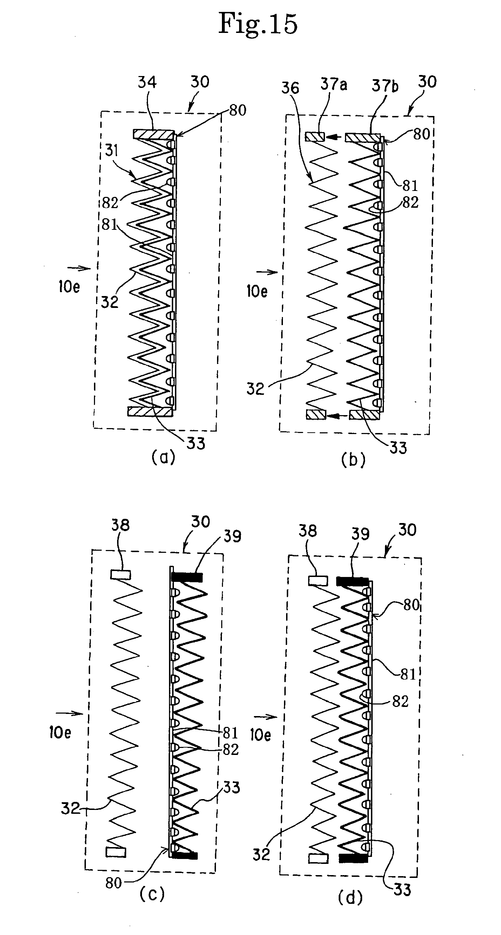

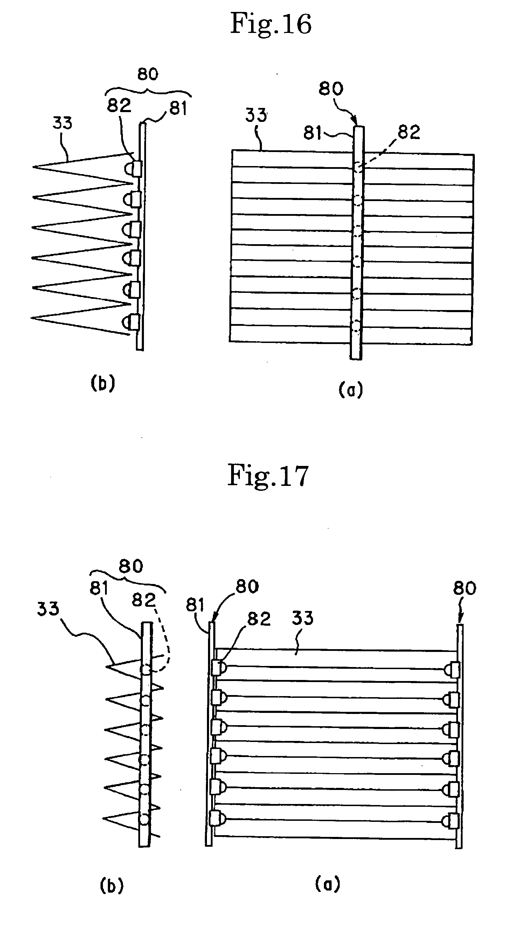

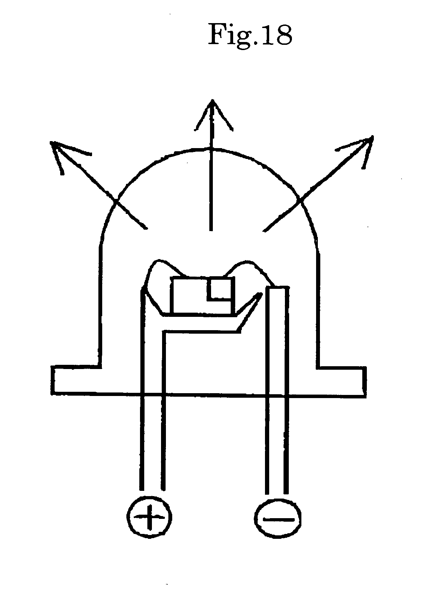

Fig. 1 is a side cross-sectional schematic view when one embodiment of the photocatalyst deodorizer according to the present invention is installed inside an air conditioner for a vehicle. Fig. 2 shows schematic plan views of example structures of the filter unit shown in Fig. 1, wherein (a) shows the case where the dust collecting filter and the deodorizing filter are connected to form one body, (b) shows the case where a filter is formed in which the dust collecting filter and the deodorizing filter are freely removable and are placed adjacent to or in contact with each other to match multiple layers, (c) shows the case where the dust collecting filter and the deodorizing filter are separate bodies, and the tubular light source is arranged therebetween, and (d) shows the case where the dust collecting filter and the deodorizing filter are separate bodies, and the tubular light source is arranged behind the filters. Fig. 3 shows schematic views of the filter unit shown in Fig. 2(b), wherein (a) is a perspective view, (b) is a front view, and (c) is a cross-sectional structural model view taken along the lines A-A' of a xenon external electrode lamp which is the tubular light source 35. Fig. 4 is a schematic view of a filter unit in which a second deodorizing filter is added and arranged in Fig. 2(b), wherein (a) is a perspective view, (b) is a front view, and (c) is a cross-sectional structural model view taken along the lines B-B' of a xenon external electrode lamp which is the tubular light source 35. Fig. 5 is a cross-sectional schematic view in the pleat intersecting direction of the dust collecting filter and the deodorizing filter processed to have a pleated shaped according to the present invention. Fig. 6 shows side schematic views when one embodiment of the photocatalyst deodorizer according to the present invention is installed inside an air conditioner for a vehicle, wherein (a) shows the case where there is one tubular light source, and (b) shows the case where there are two tubular light sources. Fig. 7 shows plan schematic views of example structures of the filter unit shown in Fig. 6(a), wherein (a) shows the case where the dust collecting filter and the deodorizing filter are connected to form one body, (b) shows the case where a filter is formed in which the dust collecting filter and the deodorizing filter are freely removable and are placed adjacent to or in contact with each other to match multiple layers, (c) shows the case where the dust collecting filter and the deodorizing filter are separate bodies, and the tubular light source is arranged therebetween, and (d) shows the case where the dust collecting filter and the deodorizing filter are separate bodies, and the tubular light source is arranged behind the deodorizing filter. Fig. 8 shows structures of the positional relationship of the tubular light source and the deodorizing filter of the filter unit, wherein (a) shows the case where a notch with an occluded opening capable of housing the tubular light source in a direction which intersects the peaks and valleys of the filter is provided in one end portion of the deodorizing filter in the direction of the peaks and valleys of the pleats, and the tubular light source is arranged inside the notch to obtain total surface irradiation of one side of the deodorizing filter, (b) shows the case where a notch with an occluded opening capable of housing the tubular light source in a direction which intersects the peaks and valleys of the pleats is provided in a center portion of the deodorizing filter in the direction of the peaks and valleys of the pleats, and the tubular light source is arranged inside the notch to obtain total surface irradiation of one side of the deodorizing filter, and (c) shows the case where a fold capable of housing the tubular light source in a direction which intersects the peaks and valleys of the pleats is provided in one end portion of the deodorizing filter in the direction of the peaks and valleys of the pleats, and the tubular light source is arranged inside the fold to obtain total surface irradiation of one side of the deodorizing filter. Fig. 9 shows a structure of the positional relationship of the tubular light source and the deodorizing filter of the filter unit, and shows the case where a notch with an occluded opening capable of housing the tubular light source in a direction which intersects the peaks and valleys of the filter is provided in both end portions of the deodorizing filter in the direction of the peaks and valleys of the pleats, and a tubular light source is arranged inside each notch to obtain total surface irradiation of one side of the deodorizing filter. Fig. 10 shows schematic views of the filter unit shown in Fig. 7(b), wherein (a) is a perspective view, (b) is a front view, and (c) is a cross-sectional structural model view taken along the lines A-A' of a xenon external electrode lamp which is the tubular light source 35. Fig. 11 shows schematic views of a filter unit in which the second deodorizing filter shown in Fig. 12(b) is added and arranged, wherein (a) is a perspective view, (b) is a front view, and (c) is a cross-sectional structural model view taken along the lines B-B' of a xenon external electrode lamp which is the tubular light source 35. Fig. 12 shows plan schematic views of example structures of a filter unit in which a second deodorizing filter is arranged in the very back, wherein (a) shows the case where the dust collecting filter and the deodorizing filter are connected to form one body, (b) shows the case where a filter is formed in which the dust collecting filter and the deodorizing filter are freely removable and are placed adjacent to or in contact with each other to match multiple layers, (c) shows the case where the dust collecting filter and the deodorizing filter are separate bodies, and the tubular light source is arranged therebetween, and (d) shows the case where the dust collecting filter and the deodorizing filter are separate bodies, and the tubular light source is arranged behind the deodorizing filter. Fig. 13 shows plan schematic views of example structures of a filter unit in which a second deodorizing filter is arranged in the very back, wherein (a) shows the case where a depression capable of housing the tubular light source is provided in the deodorizing filter side in a direction which intersects the peaks and valleys of the pleats, and the tubular light source is arranged inside the depression, (b) shows the case where a depression capable of housing the tubular light source is provided in the second deodorizing filter side in a direction which intersects the peaks and valleys of the pleats, and the tubular light source is arranged inside the depression, and (c) shows the case where a depression capable of housing the tubular light source is provided in both the deodorizing filter and the second deodorizing filter in a direction which intersects the peaks and valleys of the pleats, and the tubular light source is arranged inside the depression. Fig. 14 shows a schematic view when a photocatalyst deodorizer in which light-emitting diode elements are arranged as a light source is installed inside an air conditioner for a vehicle. Fig. 15 shows plan schematic views of example structures of the filter unit 30 shown by the dashed lines of Fig. 14 (the view is from above in Fig. 14), wherein (a) shows the case where the tubular light source in Fig. 7(a) is replaced by the light source 80, (b) shows the case where the tubular light source in Fig. 7(b) is replaced by the light source 80, (c) shows the case where the tubular light source in Fig. 7(c) is replaced by the light source 80, and (d) shows the case where the tubular light source in Fig. 7(d) is replaced by the light source 80. Fig. 16 shows schematic views of one structural arrangement of the light source which uses light-emitting diode elements and the deodorizing filter of the filter unit, wherein (a) is a schematic view showing the filter surface, and (b) is a view from the filter side surface which intersects the peaks and valleys of the pleats. Fig. 17 shows schematic views of another structural arrangement of the light source which uses light-emitting diode elements and the deodorizing filter of the filter unit, wherein (a) is a schematic view showing the filter surface, and (b) is a view from the filter side surface which intersects the peaks and valleys of the pleats. Fig. 18 is a conceptual view showing the lamp structure of an ultraviolet light-emitting diode element used in the present invention. The meaning of the reference characters is as follows. 10 is an air conditioner body, 11 is a damper, 10a is an air intake port, 10b is an inside air intake port, 10c is an outside air intake port, 10d is an air passage, 10e is an airflow, 10f is a purified air discharge port, 20 is a blower, 30 is a filter unit, 31 is a filter, 32 is a dust collecting filter, 33 is a deodorizing filter, 33a is a folding line, 34, 37a, 37b, 38 and 39 are frames, 35 is a tubular light source, 36 is a filter, 40 is an evaporator, 50 is a glass tube, 51 is an external electrode, 52 is a fluorescent material, 53 is an aperture, 60 is a second deodorizing filter, 70 is a depression formed by a notch having an occluded opening or formed by folding the pleats, 75 is a resin sheet portion which occludes the opening of a notch, 80 is a light source, 81 is a substrate, 82 is a light-emitting diode element, and 100, 200 are photocatalyst deodorizers.

### PREFERRED EMBODIMENTS OF THE INVENTION

The present invention is described in detail below, but it should not be interpreted that the present invention is limited to the embodiments described below. A photocatalyst deodorizer according to the present invention is described with reference to Figs. 1 - 18. The same reference characters are used for the same parts.

### First Embodiment

Fig. 1 shows a side schematic view when a photocatalyst deodorizer according to the present invention is installed inside an air conditioner for a vehicle. This photocatalyst deodorizer 100 is equipped with an air conditioner body 10. An air intake port 10a formed from an inside air intake port 10b and an outside air intake port 10c which are opened and closed by a damper 11, an air passage 10d connected to the air intake port 10a, and a purified air discharge port 10f are formed in the air conditioner body 10. A blower 20, a filter unit 30 and an evaporator 40 are provided in that order from the upstream side in the air passage 10d. When the blower 20 is driven, air is sucked inside the air passage 10d from the open side of the air intake port 10a. This air forms an airflow 10e which passes through the filter unit 30 and is cooled by the evaporator 40. Then, the airflow 10e passes through an air mixing door (not shown in the drawings), a heater (not shown in the drawings) and the like, and is blown out inside the vehicle.

The filter unit 30 is an air purifying portion, and is constructed from a dust collecting filter processed to have a pleated shape, a deodorizing filter processed to have a pleated shape and carrying a photocatalyst, and a tubular light source which emits light that activates the photocatalyst, and the tubular light source is arranged at a position which obtains total surface irradiation of one side of the deodorizing filter, and set at an emission angle which obtains total surface irradiation.

First, Fig. 2 will be used to describe the shape of the dust collecting filter and the deodorizing filter, and the configuration relationship of the dust collecting filter, the deodorizing filter and the tubular light source. Figs. 2(a) - (d) are plan views of the filter unit 30 shown by the dashed lines of Fig. 1, and are conceptual views showing example structures of the filter unit. Figs. 2(a) - (b) show only a side frame portion for describing the filter shape, and the top frame portion is not shown.

Fig. 2(a) is a plan view showing one structure of the filter unit, and shows the case where the dust collecting filter and the deodorizing filter are connected to form one body. The filter unit 30 is arranged with a dust collecting filter 32, a deodorizing filter 33 and a tubular light source 35 in that order toward the downstream of the airflow 10e. The dust collecting filter 32 and the deodorizing filter 33 are folded in pleated shapes having the same width and the same thickness, wherein integration is carried out to match multiple layers, and these are integrally fitted into and fixed to a rectangular frame 34 to form a filter 31. Fig. 5 is a cross-sectional schematic view in the pleat intersecting direction of the dust collecting filter and the deodorizing filter processed to have a pleated shaped according to the present invention. The frame is not shown. In this regard, as shown in Fig. 5, the width of the pleats is called the folding interval a, and the thickness of the pleats corresponds to the thickness b of the filter formed in a pleated shape. Because the dust collecting filter and the deodorizing filter which form the filter 31 form an integrated body, they can not be replaced separately, and are therefore replaced at the same time.

Fig. 2(b) is a plan view showing one structure of the filter unit, and shows the case where the dust collecting filter and the deodorizing filter are freely removable and are placed adjacent to or in contact with each other to match multiple layers. The filter unit 30 of Fig. 2(b) is arranged with the dust collecting filter 32, the deodorizing filter 33 and the tubular light source 35 in that order toward the downstream of the airflow 10e. The dust collecting filter 32 and the deodorizing filter 33 are formed by being folded in pleated shapes having the same width and the same thickness. However, the difference with Fig. 2(a) is that the dust collecting filter 32 is fitted into a frame 37a, and the deodorizing filter 33 is fitted into a frame 37b, whereby these filters are fixed to separate frames. The frame 37a and the frame 37b are integrated by a connector (not shown in the drawings) from which they can be freely removed, and the dust collecting filter 32 and the deodorizing filter 33 are placed adjacent to or in contact with each other to match multiple layers to form a filter 36. When the dust collecting filter 32 and the deodorizing filter 33 are integrated by the connector, the external appearance is similar to the case of Fig. 2(a). Further, in addition to the case where the filters are fixed using separate frames described above, the dust collecting filter 32 and the deodorizing filter 33 may be separately fitted into one frame (not shown in the drawings) in a freely removable manner so that it is possible to separately replace each filter. In either case, the dust collecting filter 32 and the deodorizing filter 33 can be replaced separately, and so long as these filters are placed adjacent to or in contact with each other to match multiple layers to form the filter 36, there is no limitation as to whether separate frames or one frame is used.

Fig. 2(c) is a plan view showing one structure of the filter unit, and shows the case where the dust collecting filter and the deodorizing filter are separate bodies, and the tubular light source is arranged therebetween. The filter unit 30 of Fig. 2(c) is arranged with the dust collecting filter 32, the tubular light source 35 and the deodorizing filter 33 in that order toward the downstream of the airflow 10e. The dust collecting filter 32 and the deodorizing filter 33 may be formed by being folded in pleated shapes having the same width and the same thickness, or may be formed by being folded in pleated shapes having different widths and different thicknesses. The dust collecting filter 32 is fitted into a frame 38, and the deodorizing filter 33 is fitted into a frame 39, whereby these filters are fixed to separate frames.

Fig. 2(d) is a plan view showing one structure of the filter unit, and shows the case where the dust collecting filter and the deodorizing filter are separate bodies, and the tubular light source is arranged behind the filters. The filter unit 30 of Fig. 2(d) is arranged with the dust collecting filter 32, the deodorizing filter 33 and the tubular light source 35 in that order toward the downstream of the airflow 10e. The filters may be formed by being folded in pleated shapes having the same width and the same thickness, or may be formed by being folded in pleated shapes having different widths and different thicknesses. The dust collecting filter 32 is fitted into a frame 38, and the deodorizing filter 33 is fitted into a frame 39, whereby these filters are fixed to separate frames. The frame 38 and the frame 39 are different from those of Fig. 2(b), and are not integrated.

With regard to the filter unit 30, Table 1 shows a comparison of the performance evaluations of the cases where each of the structures of Figs. 2(a) - (d) were made.

**[Table 1]**

| Type | Fig. 2 ( a ) type | Fig. 2 ( b ) type | Fig. 2 ( c ) type | Fig. 2 ( d ) type |
|---|---|---|---|---|
| Relationship of dust collecting filter and a deodorizing filter | Integrated / Inseparable | Stack Provision In An Integrated Body / Separable | Separate Bodies/ Separable | Separate Bodies/ Separable |
| Thickness of a dust collecting filter (mm) | 15 | 15 | 15 | 15 |
| Thickness of a deodorizing filter (mm) | 15 | 15 | 15 | 1 |
| Total filter thickness at time of Installation (mm) | 15 | 15 | 30 | 30 |
| Filter surface (mm × mm) | 216 × 200 | 216 × 200 | 216 × 200 | 216 × 200 |
| Thickness of filter unit (including light source) (mm) | 40 | 40 | 40 | 40 |
| Airflow residence ; 450 m³/h (P a) | 160 | 160 | 220 | 220 |
| Dust collecting efficiency (%) for 0.3 µm Dust | 10 | 10 | 10 | 10 |
| Dust Collecting efficiency (%) 0.5 µm Dust | 1 5 | 1 5 | 1 5 | 1 5 |
| Dust collecting efficiency (%) fine dust | 90 | 9 0 | 90 | 90 |
| Dust Collecting efficiency (%) coarse dust | 90 | 90 | 90 | 90 |
| 6g Dust load ; 450m ³/h (Pa) | 220 | 220 | 290 | 290 |

The photocatalyst deodorizer 100 according to the present invention can use any of the filter units shown in Figs. 2(a) ~ (d), but the structures of Fig. 2(a) and Fig. 2(b) are preferred because the total thickness occupied by the dust collecting filter and the deodorizing filter can be made thin. Further, the structure of Fig. 2(b) is the most preferred because each of the dust collecting filter and the deodorizing filter can be replaced in accordance with the life thereof. According to actual installation tests, in the structure of Fig. 2(a), the dust collecting function is lowered and replacement is necessary at 400 hours (corresponding to one year of use). However, the deodorizing function is 60% maintained by the action of the photocatalyst. Accordingly, the deodorizing filter which can still be used becomes useless. Further, if the photocatalyst is not activated, the deodorizing function is lowered and replacement is necessary at 400 hours (corresponding to one year of use). On the other hand, in the structure of Fig. 2(b), each of the dust collecting filter and the deodorizing filter can be replaced in accordance with the life thereof, wherein the dust collecting filter is replaced at 400 hours (corresponding to one year of use), and the deodorizing filter is replaced at 1200 hours (corresponding to three years of use). Accordingly, the structure of Fig. 2(b) makes it possible to create the special feature of extending the life of the deodorizing filter due to the photocatalyst. Namely, because of the structure that makes it possible to separately remove the dust collecting filter and the deodorizing filter, it is possible to reduce the frequency of replacement of the deodorizing filter, and this leads to a reduction of the expenditures of the owner of the vehicle.

A description will be given for the fixing of the tubular light source 35. The mounting of the tubular light source 35 will be described. In all of the structures of Figs. 2(a) - (d), the tubular light source 35 is fixed to a frame (not shown in the drawings) separate from the filter frames, and this separate frame is removably fixed to the air conditioner body 10. The fixing of the tubular light source 35 to the frame can be carried out by an example method in which both ends of the tubular light source 35 are inserted through and supported on the frame. Further, the fixing of such frame to the air conditioner body 10 can be carried out by an example method in which holding hooks (not shown in the drawings) are provided and fastened. At this time, the tubular light source 35 fixed to the separate frame together with the dust collecting filter and the deodorizing filter form the filter unit 30, namely, an air purifying portion. In any case, the tubular light source 35 in the present invention may be fixed using any mounting means so long as the tubular light source 35 can be mounted in a manner removable from the air conditioner body 10 and can be fixed near the filter surface of the deodorizing filter. In this way, maintenance or replacement of the tubular light source becomes easy.

Next, a description will be given for the fixing of the filter unit 30 to the air conditioner body 10. The filter unit 30 is formed from the dust collecting filter 32, the deodorizing filter 33, the frames 34, 37a, 37b, 38, 39 of these filters, the tubular light source 35 and the frame (not shown in the drawings) which supports the tubular light source 35, but these are integrally supported by a unit frame (not shown in the drawings), and the unit frame is preferably removably fixed to the air conditioner body 10. Each of such frames may be removably fixed to the air conditioner body 10 without using a unit frame. In order to removably fix these frames to the air conditioner body 10, holding hooks (not shown in the drawings) are provided on the frames. In this way, maintenance or replacement of each type of filter becomes easy.

Next, a description will be given for the dust collecting filter 32. The dust collecting filter 32 has a dust collecting function which catches dust in air, and while the filter material has no particular limitations, it is formed from a fiber material such as a paper material, a resin material or the like. Further, a nonwoven fabric of resin or the like is preferred. The optimum filter diameter is selected from the relationship of the dust collecting efficiency and the airflow resistance, but the present invention is not limited by the filter diameter. As for the filter diameter, the material of the frames 34, 37a, 38 has no particular limitation, but is preferably formed by a resin such as polypropylene or the like.

Next, a description will be given for the deodorizing filter 33. The deodorizing filter 33 has a function of adsorbing and removing odorants from the air that has had dust caught therefrom by the dust collecting filter 32. The filter base material is formed using a fiber material such as a paper material, a resin material or the like in the same way as the dust collecting filter. Further, a nonwoven fabric is preferred. The optimum filter diameter is selected from the relationship of the dust collecting and deodorizing efficiencies and the airflow resistance, but the present invention is not limited by the filter diameter. This filter base material includes an adsorbent powder such as activated carbon, zeolite, silica gel or the like mixed therein, and carries a photocatalyst formed from a metal oxide powder such as titanium oxide, zinc oxide or the like. The adsorbent powder is preferably activated carbon. The photocatalyst is mixed with the adsorbent, and a spray method or a method of mixing with the adsorbent or the like can be used as an example mixing method. The material of the frames 34, 37b, 39 has no particular limitation, but is preferably formed by a resin such as polypropylene or the like. By shining the ultraviolet light from the tubular light source 35 onto the photocatalyst of the deodorizing filter 33, OH radicals are created from adsorbed water. The strong oxidizing force of these OH radicals decomposes the odorants adsorbed on the adsorbent of the filter 33, whereby the absorbent is regenerated.

Next, the tubular light source 35 will be described. The tubular light source should be a light source which activates a photocatalyst, and uses a type of light source which emits light from visible light to ultraviolet light or only ultraviolet light. In particular, the tubular light source 35 can use a mercury lamp, a cathode tube (CFL), a black light, a cold cathode tube (CCFL), or a xenon external electrode lamp. The tubular light source according to the present invention indicates a lamp formed by a glass tube described above, and includes a light source which emits light in a cylindrical shape like a line light source and a light source in which light-emitting diodes are lined up.

In the present embodiment, a xenon external electrode lamp is particularly preferred for the tubular light source 35. The reason for this is that in recent years the use of mercury has decreased due to the problem of environmental pollution, and the same correspondence has also become necessary for ultraviolet lamps. In this regard, light-emitting diodes can also be used as an ultraviolet light source which does not use mercury, but because the ultraviolet light intensity of one light-emitting diode is weak, many light-emitting diodes need to be lined up. In contrast with this, a xenon external electrode lamp can not structurally achieve a 360° total angular emission, but because the emission angle of the light can be adjusted, irradiation can be carried out efficiently on only the deodorizing filter, whereby it is also possible to prevent other parts from being degraded by ultraviolet light irradiation. Table 2 shows the relationship of each type of lamp and the amount of mercury.

**[Table 2]**

| Name of Lamp | Mercury Content (mg/body) |
|---|---|
| Fluorescent lamp (Hot Cathode Tube) | 20 |
| C C F L (Cold Cathode Tube) | 2.2 |
| Light -Emitting Diode | 0 |
| Xenon External Electrode Lamp | 0 |

Further, Table 3 shows the relationship of each type of lamp and the ultraviolet light intensity.

**[Table 3 ]**

| Name of Lamp | Ultraviolet Light (360nm) Irradiation Intensity mW/ c m² (Distance : 10mm) |
|---|---|
| C C F L (Cold cathode tube) | 2.200 |
| Light-Emitting Diodes (An arrangement of 10 in a row) | 0.543 |
| Xenon External Electrode Lamp | 1.200 |

Because the xenon external electrode lamp does not use mercury and has a large ultraviolet light intensity, it is optimum as a tubular light source.

Next, with reference to Fig. 3, a description will be given for the positional relationship of the tubular light source 35 and the deodorizing filter 33 at the time when the tubular light source 35 obtains total surface irradiation of one side of the deodorizing filter 33. Fig. 3 shows schematic views of the filter unit shown in Fig. 2(b), wherein (a) is a perspective view, (b) is a front view, and (c) is a cross-sectional structural model view taken along the lines A-A' of a xenon external electrode lamp which is the tubular light source 35. Further, the dust collecting filter 32 and the deodorizing filter 33 are shown in a removed state for clarification, but they form a connected state when installed. Preferably, the tubular light source 35 is arranged so that the direction of the tube axis of the tubular light source 35 forms a cross-sectional direction of the pleats of the deodorizing filter 33. Namely, the center axis X of the tubular light source 35 is orthogonal to the folding axes Y₁, Y₂, Yₙ which form the folding lines 33a of the pleats of the deodorizing filter 33, and the tubular light source 35 and the deodorizing filter 33 are arranged so that the distance d₁ between the center axis X of the tubular light source 35 and the folding axis Y₁, the distance d₂ between the center axis X of the tubular light source 35 and the folding axis Y₂, and the distance dₙ between the center axis X of the tubular light source 35 and the folding axis Yₙ are all the same. At this time, the deodorizing filter 33 folded in a pleated shape is irradiated with light having a uniform intensity at all portions.

Further, as long as the tubular light source 35 obtains total surface irradiation of one side of the deodorizing filter 33, while maintaining the same value for all of the distance d₁, the distance d₂ and the distance dₙ, the orthogonal relationship of the center axis X and the folding axes Y₁, Y₂ , Yₙ may be canceled to approach a parallel relationship. Namely, as shown in Fig. 3(a), the tubular light source 35 may be shifted in the S direction. However, if the orthogonal relationship of the center axis X and the folding axes Y₁, Y₂, Yₙ approaches to close to a parallel relationship, the pleats of the filter will block the light, and this will create places where light does not shine in one portion of the deodorizing filter, and is therefore not preferred.

Further, while maintaining the positional relationship of the tubular light source 35 and the deodorizing filter 33 described above, the tubular light source 35 may be arranged at a center portion of the deodorizing filter 33, but arranging the tubular light source 35 near one end portion of the deodorizing filter 33 at either the upper end portion or the lower end portion like that shown in Figs. 3(a)(b) is even more preferred. This is because the tubular light source 35 will not obstruct the airflow.

Further, a description will be given for the setting of the emission angle which enables the tubular light source 35 to obtain total surface irradiation of one side of the deodorizing filter 33 in the present invention. The tubular light source 35 may include angles beyond the emission angle which obtains total surface irradiation of one side of the deodorizing filter 33, but is preferably set at an emission angle which obtains total surface irradiation of only one side of the deodorizing filter 33. This is because the ultraviolet light emitted by the tubular light source 35 will degrade other parts. In a tubular light source having a 360° total direction emission angle, reflection plates (not shown in the drawings) are preferably provided to adjust the emission angle so that a total surface irradiation is obtained only for one side of the deodorizing filter 33.

In the present invention, the length of the tubular light source is preferably roughly the same as the width of the deodorizing filter. If the length of the tubular light source is shorter than the width of the deodorizing filter, it becomes difficult to irradiate the total surface of the deodorizing filter with light, and on the other hand if the length of the tubular light source is longer than the width of the deodorizing filter, spaces will be required for the portions of the tubular light source that protrude beyond the width of the deodorizing filter, and this results in the apparatus having a large size.

A detailed description will now be given for the case where a xenon external electrode lamp is used as the tubular light source 35. In a xenon external electrode lamp, there is no electrode inside the lamp, and an external electrode is provided on the outside to discharge the internal gas. In the xenon external electrode lamp in the present invention, at least one or more electrodes are provided on the outside surface of the lamp. Fig. 3(c) shows a cross-sectional structural model view taken along the lines A-A' of the xenon external electrode lamp which is the tubular light source 35. Two external electrodes 51 are arranged parallel to the tube axis on the outside of a glass tube 50, a fluorescent material 52 which is a dielectric is applied to the inside of the glass tube, and an aperture 53 is provided by removing a portion of the fluorescent material at the position precisely between the external electrodes 51, whereby light is emitted from the aperture 53. As for the filler gas, pure xenon or a mixed gas of noble gases in which xenon is the main gas is used, and there is no need for mercury to make the fluorescent material emit light. In the xenon external electrode lamp, when a high-frequency high voltage is applied to the external electrodes 51, dielectric polarization occurs in the glass which is a dielectric, and this generates a high voltage inside the lamp. Then, discharge is started when the xenon gas in contact with the glass reaches a discharge breakdown voltage. Because the ions and electrons in the plasma immediately move respectively toward the negative electrode side and the positive electrode side, the voltage inside the plasma drops suddenly, whereby discharging is completed in a short time (approximately several tens ns). This discharge is a minute discharge having a diameter in the range of 0.1mm, but such discharges are generated many times when the discharge breakdown conditions are achieved at the glass surface between the external electrodes 51. In the case of high-frequency lighting after a series of minute discharges are generated, the electric charge of the dielectric surface inside the lamp is electrically neutralized between the glass and each external electrode 51 by the application of a reverse voltage. However, the electric charge on the internal surface of the glass of the lamp remains unchanged, and in the reverse voltage, assistance of the remaining electric charge of the internal surface of the glass is obtained and a minute discharge is generated the first time the voltage is below the discharge breakdown voltage, whereafter this process is repeated. The xenon external electrode lamp obtains a high efficiency compared to some internal electrode type lamps from the prior art.

The xenon external electrode lamp is constructed to emit light at an angle θ from the aperture 53 as described above, and the angle of the aperture 53 roughly matches the irradiation angle of the ultraviolet light of the tubular light source 35. Accordingly, in the case where a xenon external electrode lamp is used as the tubular light source 35, the angle of the aperture 53 is adjusted to match the emission angle θ which enables the tubular light source 35 to obtain total surface irradiation of one side of the deodorizing filter asshown in Figs. 3(a)(b). Further, the tubular light source 35 of Fig. 3 is arranged near one end portion of the deodorizing filter 35, but in the example case where it is arranged at a filter center portion, because the emission angle θ which obtains total surface irradiation of one side of the deodorizing filter is changed, the angle of the aperture 53 may be adjusted in accordance with that.

Fig. 4 is a schematic view of a filter unit in which a second deodorizing filter is added and arranged in Fig. 2(b), wherein (a) is a perspective view, (b) is a front view, and (c) is a cross-sectional structural model view taken along the lines B-B' of a xenon external electrode lamp which is the tubular light source 35. In addition to the structures of Figs. 2(a) - (d), the filter unit 35 preferably has a structure in which a second deodorizing filter 60 is added and arranged as shown in Fig. 4. Fig. 4(a) shows a perspective view of the case where an air purifying portion which is the filter unit 35 is constructed by arranging a dust collecting filter 32, a deodorizing filter 33, a tubular light source 35, and a second deodorizing filter 60 processed to have a pleated shape and carrying a photocatalyst in an ordered stack toward the downstream side of the airflow path 10e. The frames of the filters are not shown. The dust collecting filter 32 and the deodorizing filter 33 are shown in a removed state for clarification, but they form a connected state when installed. Because the positional relationship of the dust collecting filter 32, the deodorizing filter 33 and the tubular light source 35 is the same as for the case described in Fig. 3, a description will be given for the second deodorizing filter 60. The second deodorizing filter 60 is made of the same material as the deodorizing filter 33, carries the same photocatalyst, and is folded in a pleated shape. The width and thickness may be the same as those of the deodorizing filter 33, or they may be different. The second deodorizing filter 60 is arranged so that the folding axes y₁, y₂, yₙ which form the folding lines 60a of the pleats of the second deodorizing filter 60 are parallel with the folding axes Y₁, Y₂, Yₙ which form the folding lines 33a of the pleats of the deodorizing filter 33 as shown in Fig. 4. By arranging the second deodorizing filter 60 in this way, there is no occurrence of the situation where the pleats block light from reaching one portion of the deodorizing filter in the same manner as with the deodorizing filter 33. The center axis X of the tubular light source 35 preferably has an orthogonal relationship with Y₁, Y₂, Yₙ and Y_{1,} Y₂, Yₙ, but in the same way as with the case of Figs. 2(a) - (d) in which a second deodorizing filter 60 is not provided, it is possible to move away from the orthogonal relationship so long as the condition that the tubular light source 35 obtains total surface irradiation of one side of the deodorizing filter 33 and the condition that the tubular light source 35 obtains total surface irradiation of one side of the second deodorizing filter 60 are satisfied.

In the case where a xenon external electrode lamp is used as the tubular light source 35, an aperture 53 is provided at two places as shown in Fig. 4(c) to form a structure in which light is emitted downward in the left and right directions from the apertures 53. As shown in Figs. 3(a)(b), the left and right angles of the apertures 53 are adjusted so that they respectively match an emission angle 61 which enables the tubular light source 35 to obtain total surface irradiation of one side of the deodorizing filter 33, and an emission angle θ2 which enables the tubular light source 35 to obtain total surface irradiation of one side of the second deodorizing filter 60. The tubular light source 35 of Fig. 4 is arranged near one end portion of the deodorizing filter 33, but in the example case where it is arranged at a filter center portion, because the emission angle θ which obtains total surface irradiation of one side of the deodorizing filter 33 is changed, the angle of the aperture 53 may be adjusted in accordance with that. This is the same for the aperture 53 for irradiating the second deodorizing filter 60.

In the present embodiment, a description was given for the case of one tubular light source, but two or more tubular light sources may be provided. For example, one tubular light source may be provided at each of both end portions of the deodorizing filter. Further, in order to improve the air purifying efficiency, a plurality of filter units may be provided in the air conditioner body 10. Further, in the present embodiment, the filter unit 30 is arranged so that the tubular light source 35 is horizontal with respect to the air conditioner body 10, but it may be arranged vertically. Further, when the filters are processed to have pleated shapes, they are not limited to being folded in V shapes as shown in Figs. 2 ∼ 4, and may be processed so that peaks and valleys can be formed curvilinearly in U shapes.

### Second Embodiment

Fig. 6 shows side schematic views when a photocatalyst deodorizer according to the present invention is installed inside an air conditioner for a vehicle, wherein (a) shows the case where there is one tubular light source, and (b) shows the case where there are two tubular light sources. Except for the filter unit 30, the structure is the same as Fig. 1.

The filter unit 30 is an air purifying portion which is described in detail below using Figs. 7 - 13, and is constructed from a dust collecting filter processed to have a pleated shape, a deodorizing filter processed to have a pleated shape and carrying a photocatalyst, and a tubular light source which emits light that activates the photocatalyst, wherein a depression capable of housing the tubular light source is provided in the deodorizing filter in a direction which intersects the peaks and valleys of the pleats, and the tubular light source is arranged inside the depression to obtain total surface irradiation of one side of the deodorizing filter. The frames of the filters are not shown in Fig. 6.

Fig. 7 will be used to describe the shape of the dust collecting filter and the deodorizing filter, and the configuration relationship of the dust collecting filter, the deodorizing filter and the tubular light source. Figs. 7(a) - (d) are plan views of the filter unit 30 shown by the dashed lines of Fig. 6(a) (the view is from above in Fig. 6(a)), and are conceptual views showing example structures of the filter unit. Figs. 7(a) - (d) show only a side frame portion for describing the filter shape, and the top frame portion is not shown. Further, the configuration relationship of the dust collecting filter, the deodorizing filter and the tubular light source in the airflow direction in Fig. 7 will now be described, and the detailed positional relationship of the tubular light source and the deodorizing filter will be described later.

Fig. 7(a) is the same as Fig. 2(a) except for the fact that the tubular light source 35 is housed in a depression provided in the deodorizing filter 33, and by forming this kind of structure, the filter can be made thin, whereby this structure contributes to the miniaturization of the apparatus.

Fig. 7(b) is the same as Fig. 2(b) except for the fact that the tubular light source 35 is housed in a depression provided in the deodorizing filter 33, and this structure is the most preferred of Figs. 7(a) - (d) because the dust collecting filter and the deodorizing filter can be replaced at suitable replacement times, and because the thicknesses of these filters can be made thin, whereby the entire apparatus can be miniaturized.

Fig. 7(c) is the same as Fig. 2(c) except for the fact that the tubular light source 35 is housed in a depression provided in the deodorizing filter 33.

Fig. 7(d) is the same as Fig. 2(d) except for the fact that the tubular light source 35 is housed in a depression provided in the deodorizing filter 33, and the filters are slightly thicker compared with Figs. 7(a)(b).

With regard to the filter unit 30, Table 4 shows a comparison of the performance evaluations of the cases where each of the structures of Figs. 7(a) - (d) were made.

**[Table 4]**

| Type | Fig.7 (a) type | Fig.7 ( b ) type | Fig.7 ( c ) type | Fig.7 ( d ) type |
|---|---|---|---|---|
| Relationship of dust collecting filter and a deodorizing filter | Integrated / Inseparable | Stack Provision In An Integrated Body / Separable | Separate Bodies/ Separable | Separate Bodies/ Separable |
| Thickness of a dust collecting filter (mm) | 15 | 15 | 15 | 15 |
| Thickness of a deodorizing filter (mm) | 15 | 15 | 15 | 15 |
| Total filter thickness at time of Installation (mm) | 15 | 15 | 30 | 30 |
| Filter surface (mm × mm) | 216 × 200 | 216 × 200 | 216 × 200 | 216 × 200 |
| Thickness of filter unit (including light source) (mm) | 15 | 15 | 15 | 15 |
| Airflow residence ; 450 m³/h (P a) | 160 | 160 | 220 | 220 |
| Dust collecting efficiency (%) for 0.3 µm Dust | 10 | 10 | 10 | 10 |
| Dust Collecting efficiency (%) 0.5 µm Dust | 15 | 15 | 15 | 15 |
| Dust collecting efficiency (%) fine dust | 90 | 90 | 90 | 90 |
| Dust Collecting efficiency (%) coarse dust | 90 | 90 | 90 | 90 |
| 6g Dust load ; 450m ³/h (P a) | 220 | 220 | 290 | 290 |

The photocatalyst deodorizer 100 according to the present invention can use any of the filter units shown in Figs. 7(a) - (d) in the same way as with the case of Fig. 2. The filter units of Fig. 7 make the filter units of Fig. 2 even thinner, but have the same filter performance, and while Fig. 7(a) and Fig. 7(b) are particularly preferred, the structure of Fig. 7(b) is the most preferred because each of the dust collecting filter and the deodorizing filter can be replaced in accordance with the life thereof. In actual installation tests, Fig. 7(a) and Fig. 2(a) are the same, and Fig. 7(b) and Fig. 2(b) are the same.

Next, using Fig. 8, a description will be given for the positional relationship of the tubular light source and the deodorizing filter. Further, the frames of the filters are not shown in Fig. 8. Fig. 8(a) is a schematic view showing the case where a depression 70 capable of housing the tubular light source 35 in a direction which intersects the peaks and valleys of the pleats is provided in one end portion of the deodorizing filter 33 in the direction of the peaks and valleys of the pleats, and the tubular light source 35 is arranged inside the depression 70 to obtain total surface irradiation of one side of the deodorizing filter 33. The depression 70 of Fig. 8(a) is formed by providing a notch inclined with respect to the direction of the peaks and valleys of the filter. However, when a notch is formed, because the airflow does not flow through the filter surface and is short-circuited (bypassed), the opening is occluded by a resin film 75 or the like. Preferably, occlusion is carried out by integrally forming the resin with the filter in which the notch is formed.

Fig. 8(b) is a schematic view showing the case where a depression 70 capable of housing the tubular light source 35 in a direction which intersects the peaks and valleys of the pleats is provided in a center portion of the deodorizing filter 33 in the direction of the peaks and valleys of the pleats, and the tubular light source 35 is arranged inside the depression 70 to obtain total surface irradiation of one side of the deodorizing filter 33. The depression 70 of Fig. 8(b) is formed by providing a V-shaped notch with respect to the direction of the peaks and valleys of the filters. The opening of the notch is occluded by a resin film 75 or the like in the same way as in Fig. 8(a). Occlusion in this case is also preferably carried out by integrally forming the resin with the filter in which the notch is formed. The shape of the notch is not limited to a V shape, and may be a semicircular shape or a semi-elliptical shape.

A nonwoven fabric is applied and formed in the same shape as the filter shape having a notch like that shown in Figs. 8(a)(b), and this nonwoven fabric may be used as the deodorizing filter. This case is preferred because there is no reduction of the filter surface area due to the fact that the notch portion occluded by the resin 75 in Figs. 8(a)(b) is formed by a nonwoven fabric. The concept of the deodorizing filter of the present invention is not limited to only the case where the opening is occluded by the resin 75 or the like as in Figs. 8(a)(b), and also includes a deodorizing filter in which a notch is formed by forming the nonwoven fabric as an integrated body.

Fig. 8(c) is a schematic view showing the case where a depression 70 capable of housing the tubular light source 35 in a direction which intersects the peaks and valleys of the pleats is provided in one end portion of the deodorizing filter 33 in the direction of the peaks and valleys of the pleats, and the tubular light source 35 is arranged inside the depression 70 to obtain total surface irradiation of one side of the deodorizing filter 33. The depression 70 of Fig. 8(c) is formed by folding the pleats of the filter. The difference with Figs. 8(a)(b) is that because an opening is not formed, there is no need to carry out occlusion. Accordingly, because a filter is also formed in the depression 70, there is no lowering of the filter efficiency, and for this reason such arrangement is preferred.

The present invention is not limited to one tubular light source. For example, as shown in Fig. 9 which is the case where two tubular light sources are used, a depression 70 capable of housing a tubular light source 35 is provided in both end portions of the deodorizing filter 33 in the direction of the peaks and valleys of the pleats, and each tubular light source 35 may be arranged inside each depression 70 to obtain total surface irradiation of one side of the deodorizing filter 33. In this case, the activation of the photocatalyst will be improved because the deodorizing filter 33 will receive twice the amount of light compared with the case of Fig. 8(a). In Fig. 9, the case where the depressions 70 are notches having occluded openings is shown as an example, but the arrangement of Fig. 8(c) in which the pleats are folded to form a depression may be applied to provide a depression at both end portions of the deodorizing filter 33.

The fixing of the tubular light sources 35 is the same as that of the first embodiment in any of the arrangements shown in Figs. 7(a) - (d), Fig. 8, Fig. 9, wherein they are fixed to a frame (not shown in the drawings) separate from the filter frames and such separate frame is removably fixed to the air conditioner body 10, or they are fixed to a frame for the deodorizing filter.

The fixing of the filter unit 30 to the air conditioner body 10 is the same as that of the first embodiment.

The dust collecting filter 32 is the same as that of the first embodiment. However, in the second embodiment, a depression having a notch shape with an occluded opening or a depression formed by folding the pleats is provided in the deodorizing filter. Preferably, a depression having the same shape as the depression of the deodorizing filter is provided in the dust collecting filter at the same position in order to make the space in the direction of the thickness of the deodorizing filter and the dust collecting filter which are stacked together extremely thin, namely, so that the thickness direction when the depression of the deodorizing filter is in contact with the dust collecting filter is not prevented from being made thin. The depression provided in the dust collecting filter is preferably a depression having a notch shape with an occluded opening or a depression formed by folding the pleats in the same way as with the deodorizing filter. As for the method of occluding the opening, preferably a resin and the filter are integrally formed so that the opening of the filter is occluded by a resin film. By occluding the opening, it is possible to prevent airflow leaks, namely, airflow bypassing. Further, in the case where the filter material is made a nonwoven fabric, a nonwoven fabric is applied and formed in a shape which forms the filter shape having the notch, and this nonwoven fabric is preferably used as the dust collecting filter. In this case, because the notch portion can also be formed by a nonwoven fabric, there is no reduction of the filter surface area like that which occurs when the opening due to the notch is occluded by a resin, and therefore this case is preferred.

The deodorizing filter 33 is the same as that of the first embodiment, but in the second embodiment, a depression having a notch shape with an occluded opening or a depression formed by folding the pleats is provided in the deodorizing filter in order to secure a space for housing the tubular light source. As for the method of occluding the opening, preferably a resin and the filter are integrally formed so that the opening of the filter is occluded by a resin film. By occluding the opening, it is possible to prevent airflow leaks. Further, in the case where the filter material is made a nonwoven fabric, a nonwoven fabric is applied and formed in a shape which forms the filter shape having the notch, and this nonwoven fabric is preferably used as a dust collecting filter. In this case, because the notch portion can also be formed by a nonwoven fabric, there is no reduction of the filter surface area like that which occurs when the opening due to the notch is occluded by a resin, and therefore this case is preferred.

The tubular light source 35 is the same as that of the first embodiment.

Next, with reference to Fig. 10, a description will be given for the positional relationship of the tubular light source 35 and the deodorizing filter 33 at the time when the tubular light source 35 obtains total surface irradiation of one side of the deodorizing filter 33. Fig. 10 shows schematic views of the filter unit shown in Fig. 7(b), wherein (a) is a perspective view, (b) is a front view when Fig. 6 forms the basis, and (c) is a cross-sectional structural model view taken along the lines A-A' of a xenon external electrode lamp which is the tubular light source 35. Further, the dust collecting filter 32 and the deodorizing filter 33 are shown in a removed state for clarification, but they form a connected state when installed. The tubular light source 35 is housed inside the depression 70, and the tubular light source 35 is preferably arranged so that the direction of the tube axis of the tubular light source 35 forms a direction which intersects the peaks and valleys of the pleats of the deodorizing filter 33. The relationship of the center axis X, the peak/valley axes Y₁, Y₂, Yₙ, the distance d₁, the distance d₂, and the distance dₙ is the same as that described in the first embodiment.

Further, while maintaining this kind of positional relationship of the tubular light source 35 and the deodorizing filter 33, the tubular light source 35 may be arranged at a center portion of the deodorizing filter 33 in the direction of the peaks and valleys of the pleats as shown in Fig. 8(b). Preferably, the tubular light source 35 is arranged near one end portion of the deodorizing filter 33 at either the upper end portion or the lower end portion in the direction of the peaks and valleys of the pleats like that shown in Figs. 8(a)(b), or a tubular light source 35 is arranged at both end portions of the deodorizing filter in the direction of the peaks and valleys of the pleats as shown in Fig. 9.

The emission angle at which the tubular light source 35 obtains total surface irradiation of one side of the deodorizing filter 33 is set in the same way as in the first embodiment.

The xenon external electrode lamp used as the tubular light source 35 is the same as that of the first embodiment.

The angles θ1, θ2 of the apertures 53 are adjusted as shown in Figs. 10(a)(b) based on the same thinking as that in the first embodiment.

Fig. 11 shows schematic views of a filter unit in which a second deodorizing filter is added and arranged in Fig. 7(b), namely, the filter unit shown in Fig. 12(b), wherein (a) is a perspective view, (b) is a front view, and (c) is a cross-sectional structural model view taken along the lines B-B' of a xenon external electrode lamp which is the tubular light source 35. The filter unit 35 is preferably one of the structures shown in Figs. 12(a) - (d). Figs. 12(a) - (d) show structures in which a second deodorizing filter 60 is added to Figs. 7(a) - (d), respectively. The relationship of the dust collecting filter 32, the deodorizing filter 33 and the tubular light source 35 is the same as the case described in Fig. 7, Fig. 8 or Fig. 9, and the second deodorizing filter 60 is the same as that of the first embodiment.

Using the case of the filter unit of Fig. 12(b) as an example, a description will be given for the arrangement of the deodorizing filter, the tubular light source and the second deodorizing filter in the direction of the airflow 10e. Figs. 13(a) - (c) show schematic views of the arrangement of the deodorizing filter, the tubular light source and the second deodorizing filter in the direction of the airflow 10e. Fig. 13(a) is the same as the arrangement shown in Fig. 12(a), wherein a depression capable of housing the tubular light source is provided in the deodorizing filter in a direction which intersects the peaks and valleys of the pleats, and the tubular light source is arranged inside the depression. In Fig. 13(b), a depression capable of housing the tubular light source is provided in the second deodorizing filter in a direction which intersects the peaks and valleys of the pleats, and the tubular light source is arranged inside the depression. In Fig. 13(c), a depression capable of housing the tubular light source is provided in both the deodorizing filter and the second deodorizing filter in a direction which intersects the peaks and valleys of the pleats, and the tubular light source is arranged inside the depression. In the present invention, in the case where a second deodorizing filter is provided, any of the arrangements of Figs. 13(a) ∼ (c) may be used. The arrangement of Fig. 13(c) is more preferred because the distance between the filter surfaces of the deodorizing filter and the second deodorizing filter is made small, whereby the direction of the airflow 10e can be made thin.

In the case where a second deodorizing filter is provided, in order to irradiate the filter surface with light having a higher intensity, preferably a depression capable of housing a tubular light source is provided in both end portions in a direction which intersects the peaks and valleys of the pleats in the same way as the case shown in Fig. 9, and a tubular light source is arranged inside each depression to obtain total surface irradiation of each of the one side of the deodorizing filter and the facing one side of the second deodorizing filter. Further, the facing one side of the second deodorizing filter forms a facing relationship with the one side of the deodorizing filter which is irradiated with light, and refers to the one side of the second deodorizing filter which is irradiated with light.

In the case where a xenon external electrode lamp is used as the tubular light source 35, an aperture 53 is provided at two places as shown in Fig. 11(c) to form a structure in which light is emitted downward in the left and right directions from the apertures 53. As shown in Figs. 11(a)(b), the left and right angles of the apertures 53 are adjusted so that they respectively match an emission angle θ1 which enables the tubular light source 35 to obtain total surface irradiation of one side of the deodorizing filter 33, and an emission angle θ2 which enables the tubular light source 35 to obtain total surface irradiation of one side of the second deodorizing filter 60. The tubular light source 35 of Fig. 11 is arranged near one end portion of the deodorizing filter 33, but in the example case where it is arranged at a filter center portion, because the emission angle θ which obtains total surface irradiation of one side of the deodorizing filter 33 is changed, the angle of the aperture 53 may be adjusted in accordance with that. This is the same for the aperture 53 for irradiating the second deodorizing filter 60.

### Third Embodiment

A description will be given for the case where light-emitting diode elements are used as a light source for activating the photocatalyst. With reference to Figs. 14 - 18, a description will be given for a photocatalyst deodorizer 200 according to the present invention. Fig. 14 shows a schematic view when the photocatalyst deodorizer 200 according to the present invention is installed inside an air conditioner for a vehicle. The basic structure is the same as Fig. 1, but the difference is that a light source 80 in which light-emitting diode elements are arranged is used in place of the tubular light source in the filter unit 30.

The filter unit 30 is an air purifying portion, and is constructed in the same way as in the first embodiment from a dust collecting filter processed to have a pleated shape, a deodorizing filter processed to have a pleated shape and carrying a photocatalyst, and a light source which emits light that activates the photocatalyst. Further, because the light source 80 formed from the light-emitting diodes and a substrate is thinner than a tubular light source, there is no need to provide the depression 70 corresponding to Fig. 8(a), for example.

In the light source 80, light-emitting diode elements 82 are arranged on top of a substrate 81 at prescribed spacings, and the light-emitting diode elements 82 emit light at wavelengths which activate the photocatalyst. As was described above, the light-emitting diode elements do not contain mercury. The light-emitting diode elements can be ultraviolet light-emitting diode elements having the structure of Fig. 18, for example. These light-emitting diode elements can emit light at the wavelengths 380 - 383nm (2.3mW radiant flux, 5.4mW/sr radiant intensity) or 383 - 386nm (2.6mW radiant flux, 5.7mW/sr radiant intensity) or 386 - 389nm (3.5mW radiant flux, 7.7mW/sr radiant intensity) or the like. In this way, the photocatalyst can be activated efficiently.

Using Fig. 15, a description will be given for the configuration relationship of the dust collecting filter, the deodorizing filter and the light source. Further, except for the fact that a depression 70 corresponding to Fig. 8(a), for example, is not provided, the shapes of the dust collecting filter and the deodorizing filter are the same as those of the first embodiment. Figs. 15(a) ∼ (d) are plan views of the filter unit 30 shown by the dashed lines of Fig. 14 (the view is from above in Fig. 14), and are conceptual views showing example structures of the filter unit. Figs. 15(a) - (d) show only a side frame portion for describing the filter shape, and the top frame portion is not shown. Further, the configuration relationship of the dust collecting filter, the deodorizing filter and the light source in the airflow direction in Fig. 15 will now be described, and the detailed positional relationship of the light source and the deodorizing filter will be described later.

Fig. 15(a) is a plan view showing one structure of the filter unit in which the tubular light source in Fig. 7(a) is replaced by a light source 80. Fig. 15(b) is a plan view showing one structure of the filter unit in which the tubular light source in Fig. 7(b) is replaced by a light source 80. Fig. 15(c) is a plan view showing one structure of the filter unit in which the tubular light source in Fig. 7(c) is replaced by a light source 80. Fig. 15(d) is a plan view showing one structure of the filter unit in which the tubular light source in Fig. 7(d) is replaced by a light source 80. The photocatalyst deodorizer 200 according to the present invention can use any of the filter units shown in Figs. 15(a) - (d). The structures of Fig. 15(a) and Fig. 15(b) are preferred because the total thickness occupied by the dust collecting filter and the deodorizing filter can be made thin. Further, for the same reason given in the case of the first embodiment, the structure of Fig. 15(b) is the most preferred because each of the dust collecting filter and the deodorizing filter can be replaced in accordance with the life thereof.

Next, using Fig. 16 and Fig. 17, a description will be given for the positional relationship of the light source and the deodorizing filter. In Fig. 16 and Fig. 17, (a) shows schematic views of the filter surface, and (b) shows views from the filter side surface (left side surface) which intersects the peaks and valleys of the pleats. Further, the frames of the filters are not shown in Fig. 16 and Fig. 17. As shown in Fig. 16(a), the light source 80 has a structure in which the light-emitting diode elements 82 are arranged on top of the substrate 81 at spacings where the light-emitting diode elements 82 are arranged at each valley of the pleats of the deodorizing filter 33. Further, the light source 80 is arranged to obtain total surface irradiation of one side of the deodorizing filter 33. As shown in Fig. 16(b), because a structure is formed in which the light-emitting diode elements 82 are arranged at spacings at each valley of the pleats of the deodorizing filter 33, even though the light emission intensity of each light-emitting diode element is weak, it is possible to irradiate the deodorizing filter 33 with sufficient light for activating the photocatalyst. Further, this structure is not limited to one light source 80 in Fig. 16, and two or more light sources may be arranged to increase the amount of irradiation and create a uniform light emission to the deodorizing filter 33. Further, the light source 80 is arranged in a center portion of the deodorizing filter 33, but the arrangement location is not limited to the center portion so long as it is possible to obtain total surface irradiation of one side of the deodorizing filter 33. Further, two or more rows of light-emitting diode elements may be arranged on top of the substrate.

As shown in Fig. 17(a), a light source 80 is arranged at both end portions of the deodorizing filter 33 in the direction of the peaks and valleys of the pleats, and each light source 80 may be constructed so that the light-emitting diode elements 82 are arranged on top of the substrate 81 at spacings where the light-emitting diode elements 82 are arranged at each valley of the pleats of the deodorizing filter 33. Further, each light source 80 is arranged to obtain total surface irradiation of one side of the deodorizing filter 33. As shown in Fig. 17(b), because a structure is formed in which the light-emitting diode elements 82 are arranged at spacings at each valley of the pleats of the deodorizing filter 33, even though the light emission intensity of each light-emitting diode element is weak, it is possible to irradiate the deodorizing filter 33 with sufficient light for activating the photocatalyst. Further, two or more rows of light-emitting diode elements may be arranged on top of the substrate.

In all of the structures of Figs. 15(a) - (d), Fig. 16, Fig. 17, the light source 80 is fixed to a frame (not shown in the drawings) separate from the filter frames and such separate frame is removably fixed to the air conditioner body 10, or the light source 80 is fixed to a frame for the deodorizing filter. The fixing of the light source 80 to the frame can be carried out by an example method in which both ends of the light source 80 are inserted through and supported on the frame. Further, the fixing of such separate frame to the air conditioner body 10 can be carried out by an example method in which holding hooks (not shown in the drawings) are provided and fastened. In any case, the light source 80 in the present invention may be fixed using any mounting means so long as the light source 80 can be mounted in a manner removable from the air conditioner body 10. In this way, it becomes easy to carry out maintenance of the light source such as replacement of parts and the like.

The fixing of the filter unit 30 to the air conditioner body 10 is the same as that in the first embodiment.

The material and the filter diameter of the dust collecting filter 32 and the deodorizing filter 33 are the same as those in the first embodiment.

In the first, second and third embodiments, the apparatus can be made small and thin.

In the first, second and third embodiments, in order to improve the air purifying efficiency, a plurality of filter units may be provided in the air conditioner body 10. Further, in the first embodiment, second embodiment, the filter unit 30 is arranged so that the tubular light source 35 or the light source 80 is horizontal with respect to the air conditioner body 10, but it may be arranged vertically. Further, when the filters are processed to have pleated shapes, they are not limited to being folded in V shapes, and may be processed so that peaks and valleys can be formed curvilinearly in U shapes.

## Claims

1. A photocatalyst deodorizer, comprising :
an air purifying portion which is removably arranged in an air passage which forms an airflow from an air intake port toward a purified air discharge port, and constructed from a dust collecting filter processed to have a pleated shape, a deodorizing filter processed to have a pleated shape and carrying a photocatalyst, and a light source which emits light that activates said photocatalyst arranged in that order in a stack, or constructed from said dust collecting filter, said light source and said deodorizing filter arranged in that order in a stack;
wherein said light source is constructed from light-emitting diode elements provided in roughly a line on top of a strip-shaped substrate at spacings where said light-emitting diode elements are arranged at each valley of the pleats of said deodorizing filter, and is arranged to obtain total surface irradiation of one side of said deodorizing filter.

2. The photocatalyst deodorizer described in claim 1, wherein the pleated shape of said dust collecting filter is processed to match multiple layers with said deodorizing filter, and said dust collecting filter is arranged to be removably stacked with respect to said deodorizing filter.

3. The photocatalyst deodorizer described in claim 1 or 2, wherein the light source is arranged at both end portions of the deodorizing filter in the direction of the peaks and valleys of the pleats.

4. The photocatalyst deodorizer described in claim 1 or 2, wherein the light source is arranged in a center portion of the deodorizing filter.

5. The photocatalyst deodorizer described in claim 1 to 4, wherein the said photocatalyst deodorizer comprise a plurality of light sources
